# EUROPEAN PATENT APPLICATION

(11) **EP 0 974 647 A1**
(43) Date of publication of application: **26.01.2000**
(21) Application number: 97924309.4
(22) Date of filing: 04.06.1997
(51) Int. Cl.: C12N 1/21, C12P 13/00

(54) **PROCESS FOR PRODUCING TARGET SUBSTANCES BY FERMENTATION**

(30) Priority: 17.06.1996 JP 15557596
(71) Applicant: Ajinomoto Co., Inc., Tokyo 104 (JP)
(72) Inventor: KUWABARA, Yoko, Ajinomoto Co., Inc., Kawasaki-shi, Kanagawa-ken 210 (JP); KIMURA, Eiichiro, Ajinomoto Co., Inc., Kawasaki-shi, Kanagawa-ken 210 (JP); KAWAHARA, Yoshio, Ajinomoto Co., Inc., Kawasaki-shi, Kanagawa-ken 210 (JP); NAKAMATSU, Tsuyoshi, Ajinomoto Co., Inc., Kawasaki-shi, Kanagawa-ken 210 (JP)
(74) Representative: Strehl Schübel-Hopf & Partner
(86) International application number: JP9701886
(87) International publication number: WO9748790

(57) **Abstract**

An object resides in controlling the retaining and dissociation of a gene extrachromosomally for efficient production of an objective substance in a fermentative manner.

By culturing and growing a microorganism containing a plasmid carrying a gene disadvantageously functioning for the production of an objective enzyme and a temperature-sensitive replication origin, on which plasmid the functional gene is solely present, at a temperature at which the plasmid is replicable, and continuously culturing the microorganism at a temperature at which the plasmid is never replicable, to dissociate the plasmid from the cells and continue the culturing, the objective substance can efficiently be produced.

SEQUENCE LISTING

(1) GENERAL INFORMATION:
(i) APPLICANT:
(A) NAME: Ajinomoto Co., Inc.
(B) STREET: No. 15-1, Kyobashi 1-chome, Chuo-ku
(C) CITY: Tokyo
(E) COUNTRY: Japan
(F) POSTAL CODE (ZIP): 104
(G) TELEPHONE: (03)5250-8178
(H) TELEFAX: (03)5250-8347
(I) TELEX J22690

(ii) TITLE OF THE INVENTION: Method for Producing Objective Substance
By Fermentation Process

(iii) NUMBER OF SEQUENCES: 10

(iv) COMPUTER READABLE FORM:
(A) MEDIUM TYPE: Floppy disk
(B) COMPUTER: IBM PC compatible
(C) OPERATING SYSTEM: PC-DOS/MS-DOS

(v) CURRENT APPLICATION DATA:
APPLICATION NUMBER 97924309.4

(2) INFORMATION FOR SEQ. ID NO: 1:

(i) SEQUENCE CHARACTERISTICS:
(A) LENGTH: 2855 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Corynebacterium

(ix) FEATURE:
(A) NAME/KEY: CDS
(B) LOCATION: 359..1987

(xi) SEQUENCE DESCRIPTION: SEQ. ID NO: 1:

GATCTTGGAA CTCGACAGTT TTCACCGTCC AGTTTGGAGC GCCTGAGCTT GCAAGCTCCA 60

GCAAGTCAGC ATTAGTGGAG CCTGTCACTT TTTCGTAAAT GACCTGGCCA AAGTCACCGT 120

TTTGGAGCAA TTTTTCCTTC AGGAGCTCAA CGTTTAGCGG CTCTCTGGAT CGTGAAATGT 180

CAACGTTCAT GGAAGCCAAT GTAGTGGGGT CGCGTCGAAA AGCGCGCTTT AAGGGCGACA 240

CGCCCAAAAA GTTTTACCTT TAAAAACTAC CCGCACGCAG CACGAACCTG TTCAGTGATG 300

TAAATCACCG CGGAAATATT GTGGACGTTA CCCCCGCCTA CCGCTACGAT TTCAAAAC 358

ATG ACC ATT TCC TCA CCT TTG ATT GAC GTC GCC AAC CTT CCA GAC ATC 406

Met Thr Ile Ser Ser Pro Leu Ile Asp Val Ala Asn Leu Pro Asp Ile

1               5                   10                  15

AAC ACC ACT GCC GGC AAG ATC GCC GAC CTT AAG GCT CGC CGC GCG GAA 454

Asn Thr Thr Ala Gly Lys Ile Ala Asp Leu Lys Ala Arg Arg Ala Glu

            20                  25                  30

GCC CAT TTC CCC ATG GGT GAA AAG GCA GTA GAG AAG GTC CAC GCT GCT 502

Ala His Phe Pro Met Gly Glu Lys Ala Val Glu Lys Val His Ala Ala

        35                  40                  45

GGA CGC CTC ACT GCC CGT GAG CGC TTG GAT TAC TTA CTC GAT GAG GGC 550

Gly Arg Leu Thr Ala Arg Glu Arg Leu Asp Tyr Leu Leu Asp Glu Gly

    50                  55                  60

TCC TTC ATC GAG ACC GAT CAG CTG GCT CGC CAC CGC ACC ACC GCT TTC 598

Ser Phe Ile Glu Thr Asp Gln Leu Ala Arg His Arg Thr Thr Ala Phe

65                  70                  75                  80

GGC CTG GGC GCT AAG CGT CCT GCA ACC GAC GGC ATC GTG ACC GGC TGG 646

Gly Leu Gly Ala Lys Arg Pro Ala Thr Asp Gly Ile Val Thr Gly Trp

                85                  90                  95

GGC ACC ATT GAT GGA CGC GAA GTC TGC ATC TTC TCG CAG GAC GGC ACC 694

Gly Thr Ile Asp Gly Arg Glu Val Cys Ile Phe Ser Gln Asp Gly Thr

            100                 105                 110

GTA TTC GGT GGC GCG CTT GGT GAG GTG TAC GGC GAA AAG ATG ATC AAG 742
Val Phe Gly Gly Ala Leu Gly Glu Val Tyr Gly Glu Lys Met Ile Lys
        115                 120                 125
ATC ATG GAG CTG GCA ATC GAC ACC GGC CGC CCA TTG ATC GGT CTT TAC 790
Ile Met Glu Leu Ala Ile Asp Thr Gly Arg Pro Leu Ile Gly Leu Tyr
    130                 135                 140
GAA GGC GCT GGC GCT CGC ATT CAG GAC GGC GCT GTC TCC CTG GAC TTC 838
Glu Gly Ala Gly Ala Arg Ile Gln Asp Gly Ala Val Ser Leu Asp Phe
145                 150                 155                 160
ATT TCC CAG ACC TTC TAC CAA AAC ATT CAG GCT TCT GGC GTT ATC CCA 886
Ile Ser Gln Thr Phe Tyr Gln Asn Ile Gln Ala Ser Gly Val Ile Pro
                165                 170                 175
CAG ATC TCC GTC ATC ATG GGC GCA TGT GCA GGT GGC AAC GCT TAC GGC 934
Gln Ile Ser Val Ile Met Gly Ala Cys Ala Gly Gly Asn Ala Tyr Gly
            180                 185                 190
CCA GCC CTG ACC GAC TTC GTG GTC ATG GTG GAC AAG ACC TCC AAG ATG 982
Pro Ala Leu Thr Asp Phe Val Val Met Val Asp Lys Thr Ser Lys Met
        195                 200                 205
TTC GTT ACC GGC CCA GAC GTG ATC AAG ACC GTC ACC GGC GAG GAA ATC 1030
Phe Val Thr Gly Pro Asp Val Ile Lys Thr Val Thr Gly Glu Glu Ile
    210                 215                 220
ACC CAG GAA GAG CTT GGC GGA GCA ACC ACC CAC ATG GTG ACC GCT GGC 1078
Thr Gln Glu Glu Leu Gly Gly Ala Thr Thr His Met Val Thr Ala Gly
225                 230                 235                 240
AAC TCC CAC TAC ACC GCT GCG ACC GAT GAG GAA GCA CTG GAT TGG GTA 1126

Asn Ser His Tyr Thr Ala Ala Thr Asp Glu Glu Ala Leu Asp Trp Val
                245                 250                 255
CAG GAC CTG GTG TCC TTC CTC CCA TCC AAC AAT CGC TCT TAC ACA CCA 1174
Gln Asp Leu Val Ser Phe Leu Pro Ser Asn Asn Arg Ser Tyr Thr Pro
            260                 265                 270
CTG GAA GAC TTC GAC GAG GAA GAA GGC GGC GTT GAA GAA AAC ATC ACC 1222
Leu Glu Asp Phe Asp Glu Glu Glu Gly Gly Val Glu Glu Asn Ile Thr
        275                 280                 285
GCT GAC GAT CTG AAG CTC GAC GAG ATC ATC CCA GAT TCC GCG ACC GTT 1270
Ala Asp Asp Leu Lys Leu Asp Glu Ile Ile Pro Asp Ser Ala Thr Val
    290                 295                 300
CCT TAC GAC GTC CGC GAT GTC ATC GAA TGC CTC ACC GAC GAT GGC GAA 1318
Pro Tyr Asp Val Arg Asp Val Ile Glu Cys Leu Thr Asp Asp Gly Glu
305                 310                 315                 320
TAC CTG GAA ATC CAG GCA GAC CGC GCA GAA AAC GTT GTT ATT GCA TTC 1366
Tyr Leu Glu Ile Gln Ala Asp Arg Ala Glu Asn Val Val Ile Ala Phe
                325                 330                 335
GGC CGC ATC GAA GGC CAG TCC GTT GGA TTT GTT GCC AAC CAG CCA ACC 1414
Gly Arg Ile Glu Gly Gln Ser Val Gly Phe Val Ala Asn Gln Pro Thr
            340                 345                 350
CAG TTC GCT GGC TGC CTG GAC ATC GAC TCC TCT GAG AAG GCA GCT CGC 1462
Gln Phe Ala Gly Cys Leu Asp Ile Asp Ser Ser Glu Lys Ala Ala Arg
        355                 360                 365
TTC GTC CGC ACC TGC GAC GCG TTT AAC ATC CCA ATC GTC ATG CTT GTC 1510
Phe Val Arg Thr Cys Asp Ala Phe Asn Ile Pro Ile Val Met Leu Val

    370                 375                 380

GAC GTC CCC GGC TTC CTT CCA GGC GCA GGC CAG GAG TAT GGT GGC ATC 1558

Asp Val Pro Gly Phe Leu Pro Gly Ala Gly Gln Glu Tyr Gly Gly Ile

385                 390                 395                 400

CTG CGT CGT GGC GCA AAG CTG CTC TAC GCA TAC GGC GAA GCA ACC GTT 1606

Leu Arg Arg Gly Ala Lys Leu Leu Tyr Ala Tyr Gly Glu Ala Thr Val

                405                 410                 415

CCA AAG ATT ACC GTC ACC ATG CGT AAG GCT TAC GGC GGA GCG TAC TGC 1654

Pro Lys Ile Thr Val Thr Met Arg Lys Ala Tyr Gly Gly Ala Tyr Cys

            420                 425                 430

GTG ATG GGT TCC AAG GGC TTG GGC TCT GAC ATC AAC CTT GCA TGG CCA 1702

Val Met Gly Ser Lys Gly Leu Gly Ser Asp Ile Asn Leu Ala Trp Pro

        435                 440                 445

ACC GCA CAG ATC GCC GTC ATG GGC GCT GCT GGC GCA GTC GGA TTC ATC 1750

Thr Ala Gln Ile Ala Val Met Gly Ala Ala Gly Ala Val Gly Phe Ile

    450                 455                 460

TAC CGC AAG GAG CTC ATG GCA GCT GAT GCC AAG GGC CTC GAT ACC GTA 1798

Tyr Arg Lys Glu Leu Met Ala Ala Asp Ala Lys Gly Leu Asp Thr Val

465                 470                 475                 480

GCT CTG GCT AAG TCC TTC GAG CGC GAG TAC GAA GAC CAC ATG CTC AAC 1846

Ala Leu Ala Lys Ser Phe Glu Arg Glu Tyr Glu Asp His Met Leu Asn

                485                 490                 495

CCG TAC CAC GCT GCA GAA CGT GGC CTG ATC GAC GGC GTG ATC CTG CCA 1894

Pro Tyr His Ala Ala Glu Arg Gly Leu Ile Asp Ala Val Ile Leu Pro

            500                 505                 510

AGC GAA ACC CGC GGA CAG ATT TCC CGC AAC CTT CGC CTG CTC AAG CAC 1942
Ser Glu Thr Arg Gly Gln Ile Ser Arg Asn Leu Arg Leu Leu Lys His
        515                 520                 525
AAG AAC GTC ACT CGC CCT GCT CGC AAG CAC GGC AAC ATG CCA CTG 1987
Lys Asn Val Thr Arg Pro Ala Arg Lys His Gly Asn Met Pro Leu
    530                 535                 540
TAAATCGGCG AATCCATAAA GGTTCAAAAG AATTCAATAA GGATTCGATA AGGGTTCGAT 2047
AAGGGTTCGA TAAGGGCCGA CTTAAATGAT TGGATGTAAA GAAATACCAA TGAAAATTGG 2107
CAACTCTTTA CACCCAATCT TTAAGACATG GGGGGTGGCG CTGGGCTAAT ATAACCGGTT 2167
AGCGAAACGA TTAGTCCCTT GTTAGGGGGA TTAACCCTCG AAGTGGGTCG TATTTTGGCG 2227
TTTGTATGTT CACACAAGAA CCCTGCACAA CGCCTTCAAA GTACGTCGAC CACGACCAAG 2287
CGCATTATTC ACTCTCACCC TTCAGGATTT AGACTAAGAA ACCATGACTG CAGCACAGAC 2347
CAAACCTGAC CTCACCACCA CGGCTGGAAA GCTGTCCGAT CTTCGCTCCC GTCTTGCAGA 2407
AGCTCAAGCT CCAATGGGCG AAGCAACTGT AGAAAAAGTG CACGCTGCTG GCAGGAAGAC 2467
TGCCCGCGAA CGTATCGAGT ATTTGCTCGA TGAGGGCTCT TTCGTAGAGA TCGATGCTCT 2527
TGCTCGTCAC CGTTCCAAGA ACTTCGGCCT GGATGCCAAG CGTCCAGCTA CTGACGGTGT 2587
TGTGACTGGT TACGGCACCA TCGATGGCCG TAAGGTCTGT GTGTTCTCCC AGGACGGCGC 2647
TGTATTCGGT GGCGCTTTGG GTGAAGTTTA TGGTGAAAAG ATCGTTAAGG TTATGGATCT 2707
TGCGATCAAG ACCGGTGTGC CTTTGATCGG AATCAATGAG GGTGCTGGTG CGCGTATCCA 2767
GGAAGGTGTT GTGTCTCTGG GTCTGTACTC ACAGATTTTC TACCGCAACA CCCAGGCGTC 2827
TGGCGTTATC CCACAGATCT CTTTGATC 2855

(2) INFORMATION FOR SEQ. ID NO: 2:

(i) SEQUENCE CHARACTERISTICS:
(A) LENGTH: 543 amino acids
(B) TYPE: amino acid
(D) TOPOLOGY: linear

(ii) MOLECULE TYPE: protein

(vi) ORIGINAL SOURCE:
(A) ORGANISM: Corynebacterium

(xi) SEQUENCE DESCRIPTION: SEQ. ID NO: 2:

Met Thr Ile Ser Ser Pro Leu Ile Asp Val Ala Asn Leu Pro Asp Ile
1               5                   10                  15

Asn Thr Thr Ala Gly Lys Ile Ala Asp Leu Lys Ala Arg Arg Ala Glu
            20                  25                  30

Ala His Phe Pro Met Gly Glu Lys Ala Val Glu Lys Val His Ala Ala
        35                  40                  45

Gly Arg Leu Thr Ala Arg Glu Arg Leu Asp Tyr Leu Leu Asp Glu Gly
    50                  55                  60

Ser Phe Ile Glu Thr Asp Gln Leu Ala Arg His Arg Thr Thr Ala Phe
65                  70                  75                  80

Gly Leu Gly Ala Lys Arg Pro Ala Thr Asp Gly Ile Val Thr Gly Trp
                85                  90                  95

Gly Thr Ile Asp Gly Arg Glu Val Cys Ile Phe Ser Gln Asp Gly Thr
            100                 105                 110

Val Phe Gly Gly Ala Leu Gly Glu Val Tyr Gly Glu Lys Met Ile Lys
        115                 120                 125

Ile Met Glu Leu Ala Ile Asp Thr Gly Arg Pro Leu Ile Gly Leu Tyr
    130                 135                 140

Glu Gly Ala Gly Ala Arg Ile Gln Asp Gly Ala Val Ser Leu Asp Phe
145                 150                 155                 160

Ile Ser Gln Thr Phe Tyr Gln Asn Ile Gln Ala Ser Gly Val Ile Pro
                165                 170                 175

Gln Ile Ser Val Ile Met Gly Ala Cys Ala Gly Gly Asn Ala Tyr Gly
            180                 185                 190
Pro Ala Leu Thr Asp Phe Val Val Met Val Asp Lys Thr Ser Lys Met
        195                 200                 205
Phe Val Thr Gly Pro Asp Val Ile Lys Thr Val Thr Gly Glu Glu Ile
    210                 215                 220
Thr Gln Glu Glu Leu Gly Gly Ala Thr Thr His Met Val Thr Ala Gly
225                 230                 235                 240
Asn Ser His Tyr Thr Ala Ala Thr Asp Glu Glu Ala Leu Asp Trp Val
                245                 250                 255
Gln Asp Leu Val Ser Phe Leu Pro Ser Asn Asn Arg Ser Tyr Thr Pro
            260                 265                 270
Leu Glu Asp Phe Asp Glu Glu Glu Gly Gly Val Glu Glu Asn Ile Thr
        275                 280                 285
Ala Asp Asp Leu Lys Leu Asp Glu Ile Ile Pro Asp Ser Ala Thr Val
    290                 295                 300
Pro Tyr Asp Val Arg Asp Val Ile Glu Cys Leu Thr Asp Asp Gly Glu
305                 310                 315                 320
Tyr Leu Glu Ile Gln Ala Asp Arg Ala Glu Asn Val Val Ile Ala Phe
                325                 330                 335
Gly Arg Ile Glu Gly Gln Ser Val Gly Phe Val Ala Asn Gln Pro Thr
            340                 345                 350
Gln Phe Ala Gly Cys Leu Asp Ile Asp Ser Ser Glu Lys Ala Ala Arg
        355                 360                 365
Phe Val Arg Thr Cys Asp Ala Phe Asn Ile Pro Ile Val Met Leu Val

    370                 375                 380

Asp Val Pro Gly Phe Leu Pro Gly Ala Gly Gln Glu Tyr Gly Gly Ile

385                 390                 395                 400

Leu Arg Arg Gly Ala Lys Leu Leu Tyr Ala Tyr Gly Glu Ala Thr Val

                405                 410                 415

Pro Lys Ile Thr Val Thr Met Arg Lys Ala Tyr Gly Gly Ala Tyr Cys

            420                 425                 430

Val Met Gly Ser Lys Gly Leu Gly Ser Asp Ile Asn Leu Ala Trp Pro

        435                 440                 445

Thr Ala Gln Ile Ala Val Met Gly Ala Ala Gly Ala Val Gly Phe Ile

    450                 455                 460

Tyr Arg Lys Glu Leu Met Ala Ala Asp Ala Lys Gly Leu Asp Thr Val

465                 470                 475                 480

Ala Leu Ala Lys Ser Phe Glu Arg Glu Tyr Glu Asp His Met Leu Asn

                485                 490                 495

Pro Tyr His Ala Ala Glu Arg Gly Leu Ile Asp Ala Val Ile Leu Pro

            500                 505                 510

Ser Glu Thr Arg Gly Gln Ile Ser Arg Asn Leu Arg Leu Leu Lys His

        515                 520                 525

Lys Asn Val Thr Arg Pro Ala Arg Lys His Gly Asn Met Pro Leu

    530                 535                 540

(2) INFORMATION FOR SEQ. ID NO: 3:

(i) SEQUENCE CHARACTERISTICS:
(A) LENGTH: 1478 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Brevibacterium lactofermentum
(B) STRAIN: A12036

(ix) FEATURE:
(A) NAME/KEY: CDS
(B) LOCATION: 89..1423
(C) IDENTIFICATION METHOD: S

(xi) SEQUENCE DESCRIPTION: SEQ. ID NO: 3

GGTACCCTTT TTGTTTTGGA CACATGTAGG GTGGCCGAAA CAAAGTAATA GGACAACAAC 60

GCTCGACCGC GATTATTTTT GGAGAATC ATG ACC TCA GCA TCT GCC CCA AGC 112
Met Thr Ser Ala Ser Ala Pro Ser
1               5

TTT AAC CCC GGC AAG GGT CCC GGC TCA GCA GTC GGA ATT GCC CTT TTA 160
Phe Asn Pro Gly Lys Gly Pro Gly Ser Ala Val Gly Ile Ala Leu Leu
    10                  15                  20

GGA TTC GGA ACA GTC GGC ACT GAG GTG ATG CGT CTG ATG ACC GAG TAC 208
Gly Phe Gly Thr Val Gly Thr Glu Val Met Arg Leu Met Thr Glu Tyr
25                  30                  35                  40

GGT GAT GAA CTT GCG CAC CGC ATT GGT GGC CCA CTG GAG GTT CGT GGC 256
Gly Asp Glu Leu Ala His Arg Ile Gly Gly Pro Leu Glu Val Arg Gly
                45                  50                  55

ATT GCT GTT TCT GAT ATC TCA AAG CCA CGT GAA GGC GTT GCA CCT GAG 304
Ile Ala Val Ser Asp Ile Ser Lys Pro Arg Glu Gly Val Ala Pro Glu

            60                  65                  70

CTG CTC ACT GAG GAC GCT TTT GCA CTC ATC GAG CGC GAG GAT GTT GAC 352

Leu Leu Thr Glu Asp Ala Phe Ala Leu Ile Glu Arg Glu Asp Val Asp

        75                  80                  85

ATC GTC GTT GAG GTT ATC GGC GGC ATT GAG TAC CCA CGT GAG GTA GTT 400

Ile Val Val Glu Val Ile Gly Gly Ile Glu Tyr Pro Arg Glu Val Val

    90                  95                  100

CTC GCA GCT CTG AAG GCC GGC AAG TCT GTT GTT ACC GCC AAT AAG GCT 448

Leu Ala Ala Leu Lys Ala Gly Lys Ser Val Val Thr Ala Asn Lys Ala

105                 110                 115                 120

CTT GTT GCA GCT CAC TCT GCT GAG CTT GCT GAT GCA GCG GAA GCC GCA 496

Leu Val Ala Ala His Ser Ala Glu Leu Ala Asp Ala Ala Glu Ala Ala

                125                 130                 135

AAC GTT GAC CTG TAC TTC GAG GCT GCT GTT GCA GCC GCA ATT CCA GTG 544

Asn Val Asp Leu Tyr Phe Glu Ala Ala Val Ala Ala Ala Ile Pro Val

            140                 145                 150

GTT GGC CCA CTG CGT CGC TCC CTG GCT GGC GAT CAG ATC CAG TCT GTG 592

Val Gly Pro Leu Arg Arg Ser Leu Ala Gly Asp Gln Ile Gln Ser Val

        155                 160                 165

ATG GGC ATC GTT AAC GGC ACC ACC AAC TTC ATC TTG GAC GCC ATG GAT 640

Met Gly Ile Val Asn Gly Thr Thr Asn Phe Ile Leu Asp Ala Met Asp

    170                 175                 180

TCC ACC GGC GCT GAC TAT GCA GAT TCT TTG GCT GAG GCA ACT CGT TTG 688

Ser Thr Gly Ala Asp Tyr Ala Asp Ser Leu Ala Glu Ala Thr Arg Leu

185                 190                 195                 200

GGT TAC GCC GAA GCT GAT CCA ACT GCA GAC GTC GAA GGC CAT GAC GCC 736

Gly Tyr Ala Glu Ala Asp Pro Thr Ala Asp Val Glu Gly His Asp Ala

                205                 210                 215

GCA TCC AAG GCT GCA ATT TTG GCA TCC ATC GCT TTC CAC ACC CGT GTT 784

Ala Ser Lys Ala Ala Ile Leu Ala Ser Ile Ala Phe His Thr Arg Val

            220                 225                 230

ACC GCG GAT GAT GTG TAC TGC GAA GGT ATC AGC AAC ATC AGC GCT GCC 832

Thr Ala Asp Asp Val Tyr Cys Glu Gly Ile Ser Asn Ile Ser Ala Ala

        235                 240                 245

GAC ATT GAG GCA GCA CAG CAG GCA GGC CAC ACC ATC AAG TTG TTG GCC 880

Asp Ile Glu Ala Ala Gln Gln Ala Gly His Thr Ile Lys Leu Leu Ala

    250                 255                 260

ATC TGT GAG AAG TTC ACC AAC AAG GAA GGA AAG TCG GCT ATT TCT GCT 928

Ile Cys Glu Lys Phe Thr Asn Lys Glu Gly Lys Ser Ala Ile Ser Ala

265                 270                 275                 280

CGC GTG CAC CCG ACT CTA TTA CCT GTG TCC CAC CCA CTG GCG TCG GTA 976

Arg Val His Pro Thr Leu Leu Pro Val Ser His Pro Leu Ala Ser Val

                285                 290                 295

AAC AAG TCC TTT AAT GCA ATC TTT GTT GAA GCA GAA GCA GCT GGT CGC 1024

Asn Lys Ser Phe Asn Ala Ile Phe Val Glu Ala Glu Ala Ala Gly Arg

            300                 305                 310

CTG ATG TTC TAC GGA AAC GGT GCA GGT GGC GCG CCA ACC GCG TCT GCT 1072

Leu Met Phe Tyr Gly Asn Gly Ala Gly Gly Ala Pro Thr Ala Ser Ala

        315                 320                 325

GTG CTT GGC GAC GTC GTT GGT GCC GCA CGA AAC AAG GTG CAC GGT GGC 1120

Val Leu Gly Asp Val Val Gly Ala Ala Arg Asn Lys Val His Gly Gly
    330                 335                 340
CGT GCT CCA GGT GAG TCC ACC TAC GCT AAC CTG CCG ATC GCT GAT TTC     1168
Arg Ala Pro Gly Glu Ser Thr Tyr Ala Asn Leu Pro Ile Ala Asp Phe
345                 350                 355                 360
GGT GAG ACC ACC ACT CGT TAC CAC CTC GAC ATG GAT GTG GAA GAT CGC     1216
Gly Glu Thr Thr Thr Arg Tyr His Leu Asp Met Asp Val Glu Asp Arg
                365                 370                 375
GTG GGC GTT TTG GCT GAA TTG GCT AGC CTG TTC TCT GAG CAA GGA ATC     1264
Val Gly Val Leu Ala Glu Leu Ala Ser Leu Phe Ser Glu Gln Gly Ile
            380                 385                 390
TCC CTG CGT ACA ATC CGA CAG GAA GAG CGC GAT GAT GAT GCA CGT CTG     1312
Ser Leu Arg Thr Ile Arg Gln Glu Glu Arg Asp Asp Asp Ala Arg Leu
        395                 400                 405
ATC GTT GTC ACG CAC TCT GCG CTG GAA TCT GAT CTT TCC CGC ACC GTT     1360
Ile Val Val Thr His Ser Ala Leu Glu Ser Asp Leu Ser Arg Thr Val
    410                 415                 420
GAA CTG CTG AAG GCT AAG CCT GTT GTT AAG GCA ATC AAC AGT GTG ATC     1408
Glu Leu Leu Lys Ala Lys Pro Val Val Lys Ala Ile Asn Ser Val Ile
425                 430                 435                 440
CGC CTC GAA AGG GAC TAATTTTACT GACATGGCAA TTGAACTGAA CGTCGGTCGT     1463
Arg Leu Glu Arg Asp
                445
AAGGTTACCG TCACG                                                    1478

(2) INFORMATION FOR SEQ. ID NO: 4:

(i) SEQUENCE CHARACTERISTICS:
(A) LENGTH: 445 amino acids
(B) TYPE: amino acid
(D) TOPOLOGY: linear

(ii) MOLECULE TYPE: protein

(vi) ORIGINAL SOURCE:
(A) ORGANISM: Brevibacterium lactofermentum

(xi) SEQUENCE DESCRIPTION: SEQ. ID NO: 4:

Met Thr Ser Ala Ser Ala Pro Ser Phe Asn Pro Gly Lys Gly Pro Gly
1               5                   10                  15
Ser Ala Val Gly Ile Ala Leu Leu Gly Phe Gly Thr Val Gly Thr Glu
            20                  25                  30
Val Met Arg Leu Met Thr Glu Tyr Gly Asp Glu Leu Ala His Arg Ile
        35                  40                  45
Gly Gly Pro Leu Glu Val Arg Gly Ile Ala Val Ser Asp Ile Ser Lys
    50                  55                  60
Pro Arg Glu Gly Val Ala Pro Glu Leu Leu Thr Glu Asp Ala Phe Ala
65                  70                  75                  80
Leu Ile Glu Arg Glu Asp Val Asp Ile Val Val Glu Val Ile Gly Gly
                85                  90                  95
Ile Glu Tyr Pro Arg Glu Val Val Leu Ala Ala Leu Lys Ala Gly Lys
            100                 105                 110
Ser Val Val Thr Ala Asn Lys Ala Leu Val Ala Ala His Ser Ala Glu
        115                 120                 125
Leu Ala Asp Ala Ala Glu Ala Ala Asn Val Asp Leu Tyr Phe Glu Ala
    130                 135                 140
Ala Val Ala Ala Ala Ile Pro Val Val Gly Pro Leu Arg Arg Ser Leu
145                 150                 155                 160

Ala Gly Asp Gln Ile Gln Ser Val Met Gly Ile Val Asn Gly Thr Thr
                165                 170                 175
Asn Phe Ile Leu Asp Ala Met Asp Ser Thr Gly Ala Asp Tyr Ala Asp
            180                 185                 190
Ser Leu Ala Glu Ala Thr Arg Leu Gly Tyr Ala Glu Ala Asp Pro Thr
        195                 200                 205
Ala Asp Val Glu Gly His Asp Ala Ala Ser Lys Ala Ala Ile Leu Ala
    210                 215                 220
Ser Ile Ala Phe His Thr Arg Val Thr Ala Asp Asp Val Tyr Cys Glu
225                 230                 235                 240
Gly Ile Ser Asn Ile Ser Ala Ala Asp Ile Glu Ala Ala Gln Gln Ala
                245                 250                 255
Gly His Thr Ile Lys Leu Leu Ala Ile Cys Glu Lys Phe Thr Asn Lys
            260                 265                 270
Glu Gly Lys Ser Ala Ile Ser Ala Arg Val His Pro Thr Leu Leu Pro
        275                 280                 285
Val Ser His Pro Leu Ala Ser Val Asn Lys Ser Phe Asn Ala Ile Phe
    290                 295                 300
Val Glu Ala Glu Ala Ala Gly Arg Leu Met Phe Tyr Gly Asn Gly Ala
305                 310                 315                 320
Gly Gly Ala Pro Thr Ala Ser Ala Val Leu Gly Asp Val Val Gly Ala
                325                 330                 335
Ala Arg Asn Lys Val His Gly Gly Arg Ala Pro Gly Glu Ser Thr Tyr
            340                 345                 350
Ala Asn Leu Pro Ile Ala Asp Phe Gly Glu Thr Thr Thr Arg Tyr His

        355                 360                 365

Leu Asp Met Asp Val Glu Asp Arg Val Gly Val Leu Ala Glu Leu Ala

    370                 375                 380

Ser Leu Phe Ser Glu Gln Gly Ile Ser Leu Arg Thr Ile Arg Gln Glu

385                 390                 395                 400

Glu Arg Asp Asp Asp Ala Arg Leu Ile Val Val Thr His Ser Ala Leu

                405                 410                 415

Glu Ser Asp Leu Ser Arg Thr Val Glu Leu Leu Lys Ala Lys Pro Val

            420                 425                 430

Val Lys Ala Ile Asn Ser Val Ile Arg Leu Glu Arg Asp

        435                 440                 445

(2) INFORMATION FOR SEQ. ID NO: 5:

(i) SEQUENCE CHARACTERISTICS:

(A) LENGTH: 20 bases

(B) TYPE: nucleic acid

(C) STRANDEDNESS: single

(D) TOPOLOGY: linear

(ii) MOLECULE TYPE: other nucleic acid

(A) DESCRIPTION: /desc = "synthetic DNA"

(iv) ANTI-SENSE: NO

(xi) SEQUENCE DESCRIPTION: SEQ. ID NO: 5:

CTGGGAAGGT GAATCGAATT

(2) INFORMATION FOR SEQ. ID NO: 6:

(i) SEQUENCE CHARACTERISTICS:

(A) LENGTH: 20 bases
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iv) ANTI-SENSE: YES

(xi) SEQUENCE DESCRIPTION: SEQ. ID NO: 6:

TCCGAGGTTT GCAGAAGATC 20

(2) INFORMATION FOR SEQ. ID NO: 7:

(i) SEQUENCE CHARACTERISTICS:
(A) LENGTH: 4394 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Brevibacterium lactofermentum
(B) STRAIN: ATCC13869

(ix) FEATURE:
(A) NAME/KEY: CDS
(B) LOCATION: 443..4213
(C) IDENTIFICATION METHOD: E

(ix) FEATURE:
(A) NAME/KEY: -35 signal
(B) LOCATION: 281..287
(C) IDENTIFICATION METHOD: P

(ix) FEATURE:
(A) NAME/KEY: -10 signal
(B) LOCATION: 307..312
(C) IDENTIFICATION METHOD: S

(ix) FEATURE:
(A) NAME/KEY: RBS
(B) LOCATION: 421..428
(C) IDENTIFICATION METHOD: S

(ix) FEATURE:
(A) NAME/KEY: terminator
(B) LOCATION: 4243..4281
(C) IDENTIFICATION METHOD: S

(xi) SEQUENCE DESCRIPTION: SEQ. ID NO: 7:

GTCGACAAGC AAAATCGAAG CGGCAGCACG CCGCGTCGGA GCCTTAAACG CCATCGCCGC 60

CATCCCTGAT GGTTTCAATC ATCAAGTCGG TGAACGCGGG CGCAACCTGT CATCCGGACA 120

GCGCCAACTG ATCGCGCTGG CGCGCGCCGA ACTCATCGAG CCTTCCATCA TGCTTCTCGA 180

CGAAGCCACC TCCACCCTCG ACCCCGCCAC CGAAGCCGTT ATCCTCAACG CCTCCGATCG 240

AGTCACTAAG GGACGCACCA GCATCATCGT CGCGCACCGC TTGGCAACCG CTAAAAGGGC 300

CGACCGTATT CTTGTTGTTG AACAAGGACG TATCATTGAG GACGGATCTC ACGACGCGTT 360

GTTGTCTGCT AACGGCACCT ACGCCCGCAT GTGGCATTTA ATGGCCTGAC ACGTTATTTT 420

TAGGAGAACT GTCAACAAAT TA ATG CTA CAA CTG GGG CTT AGG CAT AAT CAG 472
Met Leu Gln Leu Gly Leu Arg His Asn Gln
1               5                   10

CCA ACG ACC AAC GTT ACA GTG GAT AAA ATA AAG CTC AAT AAA CCC TCA 520
Pro Thr Thr Asn Val Thr Val Asp Lys Ile Lys Leu Asn Lys Pro Ser
                15                  20                  25

AGA AGC AAG GAA AAG AGG CGA GTA CCT GCC GTG AGC AGC GCT AGT ACT 568
Arg Ser Lys Glu Lys Arg Arg Val Pro Ala Val Ser Ser Ala Ser Thr

            30                  35                  40
TTC GGC CAG AAT GCG TGG CTG GTA GAC GAG ATG TTC CAG CAG TTC CAG 616
Phe Gly Gln Asn Ala Trp Leu Val Asp Glu Met Phe Gln Gln Phe Gln
        45                  50                  55
AAG GAC CCC AAG TCC GTG GAC AAG GAA TGG AGA GAA CTC TTT GAG GCG 664
Lys Asp Pro Lys Ser Val Asp Lys Glu Trp Arg Glu Leu Phe Glu Ala
    60                  65                  70
CAG GGG GGA CCA AAT GCT ACC CCC GCT ACA ACA GAA GCA CAG CCT TCA 712
Gln Gly Gly Pro Asn Ala Thr Pro Ala Thr Thr Glu Ala Gln Pro Ser
75                  80                  85                  90
GCG CCC AAG GAG TCT GCG AAA CCA GCA CCA AAG GCT GCC CCT GCA GCC 760
Ala Pro Lys Glu Ser Ala Lys Pro Ala Pro Lys Ala Ala Pro Ala Ala
                95                  100                 105
AAG GCA GCA CCG CGC GTA GAA ACC AAG CCG GCC GCC AAG ACC GCC CCT 808
Lys Ala Ala Pro Arg Val Glu Thr Lys Pro Ala Ala Lys Thr Ala Pro
            110                 115                 120
AAG GCC AAG GAG TCC TCA GTG CCA CAG CAA CCT AAG CTT CCG GAG CCA 856
Lys Ala Lys Glu Ser Ser Val Pro Gln Gln Pro Lys Leu Pro Glu Pro
        125                 130                 135
GGA CAA ACC CCA ATC AGG GGT ATT TTC AAG TCC ATC GCG AAG AAC ATG 904
Gly Gln Thr Pro Ile Arg Gly Ile Phe Lys Ser Ile Ala Lys Asn Met
    140                 145                 150
GAT ATC TCC CTG GAA ATC CCA ACC GCA ACC TCG GTT CGC GAT ATG CCA 952
Asp Ile Ser Leu Glu Ile Pro Thr Ala Thr Ser Val Arg Asp Met Pro
155                 160                 165                 170

GCT CGC CTC ATG TTC GAA AAC CGC GCG ATG GTC AAC GAT CAG CTC AAG 1000

Ala Arg Leu Met Phe Glu Asn Arg Ala Met Val Asn Asp Gln Leu Lys

                175                 180                 185

CGC ACC CGC GGT GGC AAG ATC TCC TTC ACC CAC ATC ATT GGC TAC GCC 1048

Arg Thr Arg Gly Gly Lys Ile Ser Phe Thr His Ile Ile Gly Tyr Ala

            190                 195                 200

ATG GTG AAG GCA GTC ATG GCT CAC CCG GAC ATG AAC AAC TCC TAC GAC 1096

Met Val Lys Ala Val Met Ala His Pro Asp Met Asn Asn Ser Tyr Asp

        205                 210                 215

GTC ATC GAC GGC AAG CCA ACC CTG ATC GTG CCT GAG CAC ATC AAC CTG 1144

Val Ile Asp Gly Lys Pro Thr Leu Ile Val Pro Glu His Ile Asn Leu

    220                 225                 230

GGC CTT GCC ATC GAC CTT CCT CAG AAG GAC GGC TCC CGC GCA CTT GTC 1192

Gly Leu Ala Ile Asp Leu Pro Gln Lys Asp Gly Ser Arg Ala Leu Val

235                 240                 245                 250

GTA GCA GCC ATC AAG GAA ACC GAG AAG ATG AAC TTC TCC GAG TTC CTC 1240

Val Ala Ala Ile Lys Glu Thr Glu Lys Met Asn Phe Ser Glu Phe Leu

                255                 260                 265

GCA GCA TAC GAA GAC ATC GTG ACA CGC TCC CGC AAG GGC AAG CTC ACC 1288

Ala Ala Tyr Glu Asp Ile Val Thr Arg Ser Arg Lys Gly Lys Leu Thr

            270                 275                 280

ATG GAT GAC TAC CAG GGC GTT ACC GTT TCC TTG ACC AAC CCA GGT GGC 1336

Met Asp Asp Tyr Gln Gly Val Thr Val Ser Leu Thr Asn Pro Gly Gly

        285                 290                 295

ATC GGT ACC CGC CAC TCT GTC CCA CGT CTG ACC AAG GGC CAG GGC ACC 1384

Ile Gly Thr Arg His Ser Val Pro Arg Leu Thr Lys Gly Gln Gly Thr
    300                 305                 310
ATC ATC GGT GTC GGT TCC ATG GAT TAC CCA GCA GAG TTC CAG GGC GCT 1432
Ile Ile Gly Val Gly Ser Met Asp Tyr Pro Ala Glu Phe Gln Gly Ala
315                 320                 325                 330
TCC GAA GAC CGC CTT GCA GAG CTC GGC GTT GGA AAG CTT GTC ACC ATC 1480
Ser Glu Asp Arg Leu Ala Glu Leu Gly Val Gly Lys Leu Val Thr Ile
                335                 340                 345
ACC TCC ACC TAC GAT CAC CGC GTG ATC CAG GGT GCT GTG TCC GGT GAA 1528
Thr Ser Thr Tyr Asp His Arg Val Ile Gln Gly Ala Val Ser Gly Glu
            350                 355                 360
TTC CTG CGT ACC ATG TCT CGC CTG CTC ACC GAT GAT TCC TTC TGG GAT 1576
Phe Leu Arg Thr Met Ser Arg Leu Leu Thr Asp Asp Ser Phe Trp Asp
        365                 370                 375
GAG ATC TTC GAC GCA ATG AAC GTT CCT TAC ACC CCA ATG CGT TGG GCA 1624
Glu Ile Phe Asp Ala Met Asn Val Pro Tyr Thr Pro Met Arg Trp Ala
    380                 385                 390
CAG GAC GTT CCA AAC ACC GGT GTT GAT AAG AAC ACC CGC GTC ATG CAG 1672
Gln Asp Val Pro Asn Thr Gly Val Asp Lys Asn Thr Arg Val Met Gln
395                 400                 405                 410
CTC ATT GAG GCA TAC CGC TCC CGT GGA CAC CTC ATC GCT GAC ACC AAC 1720
Leu Ile Glu Ala Tyr Arg Ser Arg Gly His Leu Ile Ala Asp Thr Asn
                415                 420                 425
CCA CTT TCA TGG GTT CAG CCT GGC ATG CCA GTT CCA GAC CAC CGC GAC 1768
Pro Leu Ser Trp Val Gln Pro Gly Met Pro Val Pro Asp His Arg Asp

            430                 435                 440
CTC GAC ATC GAG ACC CAC AGC CTG ACC ATC TGG GAT CTG GAC CGT ACC 1816
Leu Asp Ile Glu Thr His Ser Leu Thr Ile Trp Asp Leu Asp Arg Thr
        445                 450                 455
TTC AGC GTC GGT GGC TTC GGC GGC AAG GAG ACC ATG ACC CTG CGC GAG 1864
Phe Ser Val Gly Gly Phe Gly Gly Lys Glu Thr Met Thr Leu Arg Glu
    460                 465                 470
GTA CTG TCC CGC CTG CGC GCT GCC TAC ACC TTG AAG GTC GGC TCC GAA 1912
Val Leu Ser Arg Leu Arg Ala Ala Tyr Thr Leu Lys Val Gly Ser Glu
475                 480                 485                 490
TAC ACC CAC ATC CTG GAC CGC GAC GAG CGC ACC TGG CTG CAG GAC CGC 1960
Tyr Thr His Ile Leu Asp Arg Asp Glu Arg Thr Trp Leu Gln Asp Arg
                495                 500                 505
CTC GAA GCC GGA ATG CCA AAG CCA ACC CAG GCA GAG CAG AAG TAC ATC 2008
Leu Glu Ala Gly Met Pro Lys Pro Thr Gln Ala Glu Gln Lys Tyr Ile
            510                 515                 520
CTG CAG AAG CTG AAC GCC GCA GAG GCT TTC GAG AAC TTC CTG CAG ACC 2056
Leu Gln Lys Leu Asn Ala Ala Glu Ala Phe Glu Asn Phe Leu Gln Thr
        525                 530                 535
AAG TAC GTC GGC CAG AAG CGC TTC TCC CTC GAA GGT GCA GAA GCT CTC 2104
Lys Tyr Val Gly Gln Lys Arg Phe Ser Leu Glu Gly Ala Glu Ala Leu
    540                 545                 550
ATC CCA CTG ATG GAC TCC GCC ATC GAC ACC GCC GCA GGC CAG GGC CTC 2152
Ile Pro Leu Met Asp Ser Ala Ile Asp Thr Ala Ala Gly Gln Gly Leu
555                 560                 565                 570

GAC GAA GTT GTC ATC GGT ATG CCA CAC CGT GGT CGC CTC AAC GTG CTG 2200
Asp Glu Val Val Ile Gly Met Pro His Arg Gly Arg Leu Asn Val Leu
                575                 580                 585
TTC AAC ATC GTG GGC AAG CCA CTG GCA TCC ATC TTC AAC GAG TTT GAA 2248
Phe Asn Ile Val Gly Lys Pro Leu Ala Ser Ile Phe Asn Glu Phe Glu
            590                 595                 600
GGC CAA ATG GAG CAG GGC CAG ATC GGT GGC TCC GGT GAC GTG AAG TAC 2296
Gly Gln Met Glu Gln Gly Gln Ile Gly Gly Ser Gly Asp Val Lys Tyr
        605                 610                 615
CAC CTC GGT TCC GAA GGC CAG CAC CTG CAG ATG TTC GGC GAC GGC GAG 2344
His Leu Gly Ser Glu Gly Gln His Leu Gln Met Phe Gly Asp Gly Glu
    620                 625                 630
ATC AAG GTC TCC CTG ACT GCT AAC CCG TCC CAC CTG GAA GCT GTT AAC 2392
Ile Lys Val Ser Leu Thr Ala Asn Pro Ser His Leu Glu Ala Val Asn
635                 640                 645                 650
CCA GTG ATG GAA GGT ATC GTC CGC GCA AAG CAG GAC TAC CTG GAC AAG 2440
Pro Val Met Glu Gly Ile Val Arg Ala Lys Gln Asp Tyr Leu Asp Lys
                655                 660                 665
GGC GTA GAC GGC AAG ACT GTT GTG CCA CTG CTG CTC CAC GGT GAC GCT 2488
Gly Val Asp Gly Lys Thr Val Val Pro Leu Leu Leu His Gly Asp Ala
            670                 675                 680
GCA TTC GCA GGC CTG GGC ATC GTG CCA GAA ACC ATC AAC CTG GCT AAG 2536
Ala Phe Ala Gly Leu Gly Ile Val Pro Glu Thr Ile Asn Leu Ala Lys
        685                 690                 695
CTG CGT GGC TAC GAC GTC GGA GGC ACC ATC CAC ATC GTG GTG AAC AAC 2584

Leu Arg Gly Tyr Asp Val Gly Gly Thr Ile His Ile Val Val Asn Asn
    700                 705                 710
CAG ATC GGC TTC ACC ACC ACC CCA GAC TCC AGC CGC TCC ATG CAC TAC 2632
Gln Ile Gly Phe Thr Thr Thr Pro Asp Ser Ser Arg Ser Met His Tyr
715                 720                 725                 730
GCA ACC GAC TAC GCC AAG GCA TTC GGC TGC CCA GTC TTC CAC GTC AAT 2680
Ala Thr Asp Tyr Ala Lys Ala Phe Gly Cys Pro Val Phe His Val Asn
                735                 740                 745
GGT GAT GAC CCA GAG GCA GTT GTC TGG GTT GGC CAG CTG GCA ACC GAG 2728
Gly Asp Asp Pro Glu Ala Val Val Trp Val Gly Gln Leu Ala Thr Glu
            750                 755                 760
TAC CGT CGT CGC TTC GGC AAG GAC GTC TTC ATC GAC CTC GTT TGC TAC 2776
Tyr Arg Arg Arg Phe Gly Lys Asp Val Phe Ile Asp Leu Val Cys Tyr
        765                 770                 775
CGC CTC CGC GGC CAC AAC GAA GCT GAT GAT CCT TCC ATG ACC CAG CCA 2824
Arg Leu Arg Gly His Asn Glu Ala Asp Asp Pro Ser Met Thr Gln Pro
    780                 785                 790
AAG ATG TAT GAG CTC ATC ACC GGC CGC GAG ACC GTT CGT GCT CAG TAC 2872
Lys Met Tyr Glu Leu Ile Thr Gly Arg Glu Thr Val Arg Ala Gln Tyr
795                 800                 805                 810
ACC GAA GAC CTG CTC GGA CGT GGA GAC CTC TCC AAC GAA GAT GCA GAA 2920
Thr Glu Asp Leu Leu Gly Arg Gly Asp Leu Ser Asn Glu Asp Ala Glu
                815                 820                 825
GCA GTC GTC CGC GAC TTC CAC GAC CAG ATG GAA TCT GTG TTC AAC GAA 2968
Ala Val Val Arg Asp Phe His Asp Gln Met Glu Ser Val Phe Asn Glu

            830                 835                 840
GTC AAG GAA GGC GGC AAG AAG CAG GCT GAG GCA CAG ACC GGC ATC ACC 3016
Val Lys Glu Gly Gly Lys Lys Gln Ala Glu Ala Gln Thr Gly Ile Thr
        845                 850                 855
GGC TCC CAG AAG CTT CCA CAC GGC CTT GAG ACC AAC ATC TCC CGT GAA 3064
Gly Ser Gln Lys Leu Pro His Gly Leu Glu Thr Asn Ile Ser Arg Glu
    860                 865                 870
GAG CTC CTG GAA CTG GGA CAG GCT TTC GCC AAC ACC CCA GAA GGC TTC 3112
Glu Leu Leu Glu Leu Gly Gln Ala Phe Ala Asn Thr Pro Glu Gly Phe
875                 880                 885                 890
AAC TAC CAC CCA CGT GTG GCT CCA GTT GCT AAG AAG CGC GTC TCC TCT 3160
Asn Tyr His Pro Arg Val Ala Pro Val Ala Lys Lys Arg Val Ser Ser
                895                 900                 905
GTC ACC GAA GGT GGC ATC GAC TGG GCA TGG GGC GAG CTC CTC GCC TTC 3208
Val Thr Glu Gly Gly Ile Asp Trp Ala Trp Gly Glu Leu Leu Ala Phe
            910                 915                 920
GGT TCC CTG GCT AAC TCC GGC CGC TTG GTT CGC CTT GCA GGT GAA GAT 3256
Gly Ser Leu Ala Asn Ser Gly Arg Leu Val Arg Leu Ala Gly Glu Asp
        925                 930                 935
TCC CGC CGC GGT ACC TTC ACC CAG CGC CAC GCA GTT GCC ATC GAC CCA 3304
Ser Arg Arg Gly Thr Phe Thr Gln Arg His Ala Val Ala Ile Asp Pro
    940                 945                 950
GCG ACC GCT GAA GAG TTC AAC CCA CTC CAC GAG CTT GCA CAG TCC AAG 3352
Ala Thr Ala Glu Glu Phe Asn Pro Leu His Glu Leu Ala Gln Ser Lys
955                 960                 965                 970

GGC AAC AAC GGT AAG TTC CTG GTC TAC AAC TCC GCA CTG ACC GAG TAC     3400

Gly Asn Asn Gly Lys Phe Leu Val Tyr Asn Ser Ala Leu Thr Glu Tyr

                975                 980                 985

GCA GGC ATG GGC TTC GAG TAC GGC TAC TCC GTA GGA AAC GAA GAC TCC     3448

Ala Gly Met Gly Phe Glu Tyr Gly Tyr Ser Val Gly Asn Glu Asp Ser

            990                 995                 1000

GTC GTT GCA TGG GAA GCA CAG TTC GGC GAC TTC GCC AAC GGC GCT CAG     3496

Val Val Ala Trp Glu Ala Gln Phe Gly Asp Phe Ala Asn Gly Ala Gln

        1005                1010                1015

ACC ATC ATC GAT GAG TAC GTC TCC TCA GGC GAA GCT AAG TGG GGC CAG     3544

Thr Ile Ile Asp Glu Tyr Val Ser Ser Gly Glu Ala Lys Trp Gly Gln

    1020                1025                1030

ACC TCC AAG CTG ATC CTT CTG CTG CCT CAC GGC TAC GAA GGC CAG GGC     3592

Thr Ser Lys Leu Ile Leu Leu Leu Pro His Gly Tyr Glu Gly Gln Gly

1035                1040                1045                1050

CCA GAC CAC TCT TCC GCA CGT ATC GAG CGC TTC CTG CAG CTG TGC GCT     3640

Pro Asp His Ser Ser Ala Arg Ile Glu Arg Phe Leu Gln Leu Cys Ala

                1055                1060                1065

GAG GGT TCC ATG ACT GTT GCT CAG CCA TCC ACC CCA GCA AAC CAC TTC     3688

Glu Gly Ser Met Thr Val Ala Gln Pro Ser Thr Pro Ala Asn His Phe

            1070                1075                1080

CAC CTG CTG CGT CGT CAC GCT CTG TCC GAC CTG AAG CGT CCA CTG GTT     3736

His Leu Leu Arg Arg His Ala Leu Ser Asp Leu Lys Arg Pro Leu Val

        1085                1090                1095

ATC TTC ACC CCG AAG TCC ATG CTG CGT AAC AAG GCT GCT GCC TCC GCA     3784

Ile Phe Thr Pro Lys Ser Met Leu Arg Asn Lys Ala Ala Ala Ser Ala
    1100                1105                1110
CCA GAA GAC TTC ACT GAG GTC ACC AAG TTC CAA TCC GTG ATC GAC GAT 3832
Pro Glu Asp Phe Thr Glu Val Thr Lys Phe Gln Ser Val Ile Asp Asp
1115                1120                1125                1130
CCA AAC GTT GCA GAT GCA GCC AAG GTG AAG AAG GTC ATG CTG GTC TCC 3880
Pro Asn Val Ala Asp Ala Ala Lys Val Lys Lys Val Met Leu Val Ser
                1135                1140                1145
GGC AAG CTG TAC TAC GAA TTG GCA AAG CGC AAG GAG AAG GAC GGA CGC 3928
Gly Lys Leu Tyr Tyr Glu Leu Ala Lys Arg Lys Glu Lys Asp Gly Arg
            1150                1155                1160
GAC GAC ATC GCG ATC GTT CGT ATC GAA ATG CTC CAC CCA ATT CCG TTC 3976
Asp Asp Ile Ala Ile Val Arg Ile Glu Met Leu His Pro Ile Pro Phe
        1165                1170                1175
AAC CGC ATC TCC GAG GCT CTT GCC GGC TAC CCT AAC GCT GAG GAA GTC 4024
Asn Arg Ile Ser Glu Ala Leu Ala Gly Tyr Pro Asn Ala Glu Glu Val
    1180                1185                1190
CTC TTC GTT CAG GAT GAG CCA GCA AAC CAG GGC CCA TGG CCG TTC TAC 4072
Leu Phe Val Gln Asp Glu Pro Ala Asn Gln Gly Pro Trp Pro Phe Tyr
1195                1200                1205                1210
CAG GAG CAC CTC CCA GAG CTG ATC CCG AAC ATG CCA AAG ATG CGC CGC 4120
Gln Glu His Leu Pro Glu Leu Ile Pro Asn Met Pro Lys Met Arg Arg
                1215                1220                1225
GTT TCC CGC CGC GCT CAG TCC TCC ACC GCA ACT GGT GTT GCT AAG GTG 4168
Val Ser Arg Arg Ala Gln Ser Ser Thr Ala Thr Gly Val Ala Lys Val

1230                    1235                    1240

CAC CAG CTG GAG GAG AAG CAG CTT ATC GAC GAG GCT TTC GAG GCT          4213

His Gln Leu Glu Glu Lys Gln Leu Ile Asp Glu Ala Phe Glu Ala

        1245                    1250                    1255

TAAGTCTTTA TAGTCCTGCA CTAGCCTAGA GGGCCTTATG CAGTGTGAAT CACACAGCAT    4273

AAGGCCCTTT TTGCTGCCGT GGTTGCCTAA GGTGGAAGGC ATGAAACGAA TCTGTGCGGT    4333

CACGATCTCT TCAGTACTTT TGCTAAGTGG CTGCTCCTCC ACTTCCACCA CGCAGCTCGA    4393

G                                                                    4394

(2) INFORMATION FOR SEQ. ID NO: 8:

(i) SEQUENCE CHARACTERISTICS:
(A) LENGTH: 1257
(B) TYPE: amino acid
(D) TOPOLOGY: linear

(ii) MOLECULE TYPE: protein

(xi) SEQUENCE DESCRIPTION: SEQ. ID NO: 8:

Met Leu Gln Leu Gly Leu Arg His Asn Gln Pro Thr Thr Asn Val Thr

1               5                   10                  15

Val Asp Lys Ile Lys Leu Asn Lys Pro Ser Arg Ser Lys Glu Lys Arg

            20                  25                  30

Arg Val Pro Ala Val Ser Ser Ala Ser Thr Phe Gly Gln Asn Ala Trp

        35                  40                  45

Leu Val Asp Glu Met Phe Gln Gln Phe Gln Lys Asp Pro Lys Ser Val

    50                  55                  60

Asp Lys Glu Trp Arg Glu Leu Phe Glu Ala Gln Gly Gly Pro Asn Ala

65                  70                  75                  80

Thr Pro Ala Thr Thr Glu Ala Gln Pro Ser Ala Pro Lys Glu Ser Ala

                85                  90                  95

Lys Pro Ala Pro Lys Ala Ala Pro Ala Ala Lys Ala Ala Pro Arg Val

            100                 105                 110

Glu Thr Lys Pro Ala Ala Lys Thr Ala Pro Lys Ala Lys Glu Ser Ser

        115                 120                 125

Val Pro Gln Gln Pro Lys Leu Pro Glu Pro Gly Gln Thr Pro Ile Arg

    130                 135                 140

Gly Ile Phe Lys Ser Ile Ala Lys Asn Met Asp Ile Ser Leu Glu Ile

145                 150                 155                 160

Pro Thr Ala Thr Ser Val Arg Asp Met Pro Ala Arg Leu Met Phe Glu

                165                 170                 175

Asn Arg Ala Met Val Asn Asp Gln Leu Lys Arg Thr Arg Gly Gly Lys

            180                 185                 190

Ile Ser Phe Thr His Ile Ile Gly Tyr Ala Met Val Lys Ala Val Met

        195                 200                 205

Ala His Pro Asp Met Asn Asn Ser Tyr Asp Val Ile Asp Gly Lys Pro

    210                 215                 220

Thr Leu Ile Val Pro Glu His Ile Asn Leu Gly Leu Ala Ile Asp Leu

225                 230                 235                 240

Pro Gln Lys Asp Gly Ser Arg Ala Leu Val Val Ala Ala Ile Lys Glu

                245                 250                 255

Thr Glu Lys Met Asn Phe Ser Glu Phe Leu Ala Ala Tyr Glu Asp Ile

            260                 265                 270

Val Thr Arg Ser Arg Lys Gly Lys Leu Thr Met Asp Asp Tyr Gln Gly
        275                 280                 285
Val Thr Val Ser Leu Thr Asn Pro Gly Gly Ile Gly Thr Arg His Ser
    290                 295                 300
Val Pro Arg Leu Thr Lys Gly Gln Gly Thr Ile Ile Gly Val Gly Ser
305                 310                 315                 320
Met Asp Tyr Pro Ala Glu Phe Gln Gly Ala Ser Glu Asp Arg Leu Ala
                325                 330                 335
Glu Leu Gly Val Gly Lys Leu Val Thr Ile Thr Ser Thr Tyr Asp His
            340                 345                 350
Arg Val Ile Gln Gly Ala Val Ser Gly Glu Phe Leu Arg Thr Met Ser
        355                 360                 365
Arg Leu Leu Thr Asp Asp Ser Phe Trp Asp Glu Ile Phe Asp Ala Met
    370                 375                 380
Asn Val Pro Tyr Thr Pro Met Arg Trp Ala Gln Asp Val Pro Asn Thr
385                 390                 395                 400
Gly Val Asp Lys Asn Thr Arg Val Met Gln Leu Ile Glu Ala Tyr Arg
                405                 410                 415
Ser Arg Gly His Leu Ile Ala Asp Thr Asn Pro Leu Ser Trp Val Gln
            420                 425                 430
Pro Gly Met Pro Val Pro Asp His Arg Asp Leu Asp Ile Glu Thr His
        435                 440                 445
Ser Leu Thr Ile Trp Asp Leu Asp Arg Thr Phe Ser Val Gly Gly Phe
    450                 455                 460
Gly Gly Lys Glu Thr Met Thr Leu Arg Glu Val Leu Ser Arg Leu Arg

465                 470                 475                 480
Ala Ala Tyr Thr Leu Lys Val Gly Ser Glu Tyr Thr His Ile Leu Asp
                485                 490                 495
Arg Asp Glu Arg Thr Trp Leu Gln Asp Arg Leu Glu Ala Gly Met Pro
            500                 505                 510
Lys Pro Thr Gln Ala Glu Gln Lys Tyr Ile Leu Gln Lys Leu Asn Ala
        515                 520                 525
Ala Glu Ala Phe Glu Asn Phe Leu Gln Thr Lys Tyr Val Gly Gln Lys
    530                 535                 540
Arg Phe Ser Leu Glu Gly Ala Glu Ala Leu Ile Pro Leu Met Asp Ser
545                 550                 555                 560
Ala Ile Asp Thr Ala Ala Gly Gln Gly Leu Asp Glu Val Val Ile Gly
                565                 570                 575
Met Pro His Arg Gly Arg Leu Asn Val Leu Phe Asn Ile Val Gly Lys
            580                 585                 590
Pro Leu Ala Ser Ile Phe Asn Glu Phe Glu Gly Gln Met Glu Gln Gly
        595                 600                 605
Gln Ile Gly Gly Ser Gly Asp Val Lys Tyr His Leu Gly Ser Glu Gly
    610                 615                 620
Gln His Leu Gln Met Phe Gly Asp Gly Glu Ile Lys Val Ser Leu Thr
625                 630                 635                 640
Ala Asn Pro Ser His Leu Glu Ala Val Asn Pro Val Met Glu Gly Ile
                645                 650                 655
Val Arg Ala Lys Gln Asp Tyr Leu Asp Lys Gly Val Asp Gly Lys Thr
            660                 665                 670

Val Val Pro Leu Leu Leu His Gly Asp Ala Ala Phe Ala Gly Leu Gly
        675                 680                 685
Ile Val Pro Glu Thr Ile Asn Leu Ala Lys Leu Arg Gly Tyr Asp Val
    690                 695                 700
Gly Gly Thr Ile His Ile Val Val Asn Asn Gln Ile Gly Phe Thr Thr
705                 710                 715                 720
Thr Pro Asp Ser Ser Arg Ser Met His Tyr Ala Thr Asp Tyr Ala Lys
                725                 730                 735
Ala Phe Gly Cys Pro Val Phe His Val Asn Gly Asp Asp Pro Glu Ala
            740                 745                 750
Val Val Trp Val Gly Gln Leu Ala Thr Glu Tyr Arg Arg Arg Phe Gly
        755                 760                 765
Lys Asp Val Phe Ile Asp Leu Val Cys Tyr Arg Leu Arg Gly His Asn
    770                 775                 780
Glu Ala Asp Asp Pro Ser Met Thr Gln Pro Lys Met Tyr Glu Leu Ile
785                 790                 795                 800
Thr Gly Arg Glu Thr Val Arg Ala Gln Tyr Thr Glu Asp Leu Leu Gly
                805                 810                 815
Arg Gly Asp Leu Ser Asn Glu Asp Ala Glu Ala Val Val Arg Asp Phe
            820                 825                 830
His Asp Gln Met Glu Ser Val Phe Asn Glu Val Lys Glu Gly Gly Lys
        835                 840                 845
Lys Gln Ala Glu Ala Gln Thr Gly Ile Thr Gly Ser Gln Lys Leu Pro
    850                 855                 860
His Gly Leu Glu Thr Asn Ile Ser Arg Glu Glu Leu Leu Glu Leu Gly

865                 870                 875                 880

Gln Ala Phe Ala Asn Thr Pro Glu Gly Phe Asn Tyr His Pro Arg Val

                885                 890                 895

Ala Pro Val Ala Lys Lys Arg Val Ser Ser Val Thr Glu Gly Gly Ile

            900                 905                 910

Asp Trp Ala Trp Gly Glu Leu Leu Ala Phe Gly Ser Leu Ala Asn Ser

        915                 920                 925

Gly Arg Leu Val Arg Leu Ala Gly Glu Asp Ser Arg Arg Gly Thr Phe

    930                 935                 940

Thr Gln Arg His Ala Val Ala Ile Asp Pro Ala Thr Ala Glu Glu Phe

945                 950                 955                 960

Asn Pro Leu His Glu Leu Ala Gln Ser Lys Gly Asn Asn Gly Lys Phe

                965                 970                 975

Leu Val Tyr Asn Ser Ala Leu Thr Glu Tyr Ala Gly Met Gly Phe Glu

            980                 985                 990

Tyr Gly Tyr Ser Val Gly Asn Glu Asp Ser Val Val Ala Trp Glu Ala

        995                 1000                1005

Gln Phe Gly Asp Phe Ala Asn Gly Ala Gln Thr Ile Ile Asp Glu Tyr

    1010                1015                1020

Val Ser Ser Gly Glu Ala Lys Trp Gly Gln Thr Ser Lys Leu Ile Leu

1025                1030                1035                1040

Leu Leu Pro His Gly Tyr Glu Gly Gln Gly Pro Asp His Ser Ser Ala

                1045                1050                1055

Arg Ile Glu Arg Phe Leu Gln Leu Cys Ala Glu Gly Ser Met Thr Val

            1060                1065                1070

Ala Gln Pro Ser Thr Pro Ala Asn His Phe His Leu Leu Arg Arg His
        1075                1080                1085
Ala Leu Ser Asp Leu Lys Arg Pro Leu Val Ile Phe Thr Pro Lys Ser
    1090                1095                1100
Met Leu Arg Asn Lys Ala Ala Ala Ser Ala Pro Glu Asp Phe Thr Glu
1105                1110                1115                1120
Val Thr Lys Phe Gln Ser Val Ile Asp Asp Pro Asn Val Ala Asp Ala
                1125                1130                1135
Ala Lys Val Lys Lys Val Met Leu Val Ser Gly Lys Leu Tyr Tyr Glu
            1140                1145                1150
Leu Ala Lys Arg Lys Glu Lys Asp Gly Arg Asp Asp Ile Ala Ile Val
        1155                1160                1165
Arg Ile Glu Met Leu His Pro Ile Pro Phe Asn Arg Ile Ser Glu Ala
    1170                1175                1180
Leu Ala Gly Tyr Pro Asn Ala Glu Glu Val Leu Phe Val Gln Asp Glu
1185                1190                1195                1200
Pro Ala Asn Gln Gly Pro Trp Pro Phe Tyr Gln Glu His Leu Pro Glu
                1205                1210                1215
Leu Ile Pro Asn Met Pro Lys Met Arg Arg Val Ser Arg Arg Ala Gln
            1220                1225                1230
Ser Ser Thr Ala Thr Gly Val Ala Lys Val His Gln Leu Glu Glu Lys
        1235                1240                1245
Gln Leu Ile Asp Glu Ala Phe Glu Ala
    1250                1255

(2) INFORMATION FOR SEQ. ID NO: 9:

(i) SEQUENCE CHARACTERISTICS:
(A) LENGTH: 20 bases
(B) TYPE: nucleic acid
(C) STRANDEDNESS: single
(D) TOPOLOGY: linear

(ii) MOLECOLE TYPE: other nucleic acid
(A) DESCRIPTION: /desc = "synthetic DNA"

(iv) ANTI-SENSE: NO

(xi) SEQUENCE DESCRIPTION: SEQ. ID NO: 9:

CTGTCTGAAG GATCGGTTCT 20

(2) INFORMATION FOR SEQ. ID NO: 10:

(i) SEQUENCE CHARACTERISTICS:
(A) LENGTH: 29 bases
(B) TYPE: nucleic acid
(C) STRANDEDNESS: single
(D) TOPOLOGY: linear

(ii) MOLECOLE TYPE: other nucleic acid
(A) DESCRIPTION: /desc = "synthetic DNA"

(iv) ANTI-SENSE: YES

(xi) SEQUENCE DESCRIPTION: SEQ. ID NO: 10:

GAGTGCTCAG GCCCCTGTCC CTCGTAACC 29

## Description

### Technical Field of the Invention

The present invention relates to a method for producing an objective substance; more specifically, the present invention relates to a method for producing useful substances such as amino acid at a high efficiency.

### Background Art

So as to elevate the productivity of an objective substance by a fermentation process using microorganisms, a method has been known, comprising using enzymes disadvantageously working for the production of the objective substance, for example, an enzyme decomposing the objective substance or converting the objective substance into a different substance or using microorganisms wherein enzymes and the like belonging to another pathway branching from the biosynthetic pathway of the objective substance are defective or using attenuated microorganisms.

As an L-lysine-producing bacterium of Corynebacterium glutamicum, for example, a mutant strain defective in homoserine dehydrogenase (referred to as "HD" hereinbelow) has been known, which is said to have the most serious influence on the production of L-lysine [Nakayama, K. et al.; J. Gen. Appl. Microbiol. 7(3), 145-154 (1961)]. The mutant strain is defective in HD catalyzing the reaction for producing L-homoserine from aspartate β-semialdehyde as the first reaction in the L-threonine-intrinsic synthetic pathway branching through aspartate β-semialdehyde from the L-lysine synthetic pathway, so no L-threonine is synthesized. As a result, L-lysine synthesis is progressed, with no inhibition of aspartokinase activity which is under the influence of the feedback inhibition with L-threonine.

Additionally, the present inventor has transformed an L-glutamic acid-producing coryneform bacterium with a plasmid carrying α-ketoglutaric acid dehydrogenase (referred to as "α-KGDH" hereinbelow) gene, to examine the level of α-KGDH activity of the resulting transformant and the L-glutamic acid production potency thereof. Consequently, the inventor has found that the quantity of produced L-glutamic acid is reduced in the strain with an enhanced α-KGDH activity. Furthermore, the inventor has found that an L-glutamic acid-producing coryneform bacterium with the damaged α-KGDH gene produces and accumulates a prominent level of L-glutamic acid when cultured in a culture medium containing an excess of biotin, with no supplement of substances suppressing the actions of biotin, such as detergents and penicillin (the pamphlet of the International Publication WO 95/34672). It has been known that when cultured in culture media under regulations of the biotin level, generally, L-glutamic acid-producing coryneform bacteria produce distinctive quantities of L-glutamic acid, but when cultured in culture media containing an excess of biotin, these bacteria never produce L-glutamic acid. So as to produce L-glutamic acid in the presence of an excess of biotin, essentially, detergents or penicillin is to be added to the culture media. However, the bacterial strain with the damaged α-KGDH gene produces L-glutamic acid, even without any addition of such substances (the pamphlet of the International Publication WO 95/34672).

Still furthermore, the present inventor has made research works regarding through what type of action mechanism the biotin regulation or the addition of detergents or penicillin may have influence over the production of L-glutamic acid by coryneform bacteria, and consequently, the inventor has demonstrated the presence of a gene possibly involved in the production of L-glutamic acid (the gene is referred to as "'dtsR gene" hereinbelow; the protein encoded by the gene is referred to as "DTSR protein" hereinbelow). Then, the inventor has confirmed that the strain with the dtsR gene damaged produces a prominent level of L-glutamic acid, even under a condition of the presence of biotin at a level where the wild type strain can hardly produce L-glutamic acid (the pamphlet of the International Publication of WO 95/23224).

As has been described above, the productivity of an objective substance can be elevated by allowing a specific gene to be defective, but meanwhile, such gene defection may sometimes be disadvantageous for microbial growth. Because the HD-defective strain cannot synthetically produce L-threonine or L-methionine, for example, the HD-defective strain cannot grow in culture media in the absence of these amino acids. Additionally, the DTSR protein-defective strain has a high production potency of L-glutamic acid, but grows at a slow rate, and for culturing the strain, oleic acid is required (the pamphlet of the International Publication WO 95/23224). Therefore, the strain is essentially cultured in culture media to which oleic acid or derivatives thereof are added, but the addition of oleic acid or derivatives thereof not only escalates the raw material cost but also disadvantageously affects the growth of the DTSR protein-defective strain because oleic acid or derivatives thereof per se have an action suppressing the growth. Compared with the wild type strain, furthermore, the growth of the α-KGDH-defective strain is poor, disadvantageously.

From the respect of microbial growth, accordingly, the genes described above are preferably retained within cells. A method has been known, for allowing a chromosome to retain a specific gene during the growth of bacteria and to dissociate the gene therefrom for the production of an objective substance. For example, a method for preparing phenylalanine in Escherichia coli is disclosed, comprising growing the bacterium and then dissociating a gene involved in the tyrosine synthesis from the chromosome by utilizing the temperature-sensitive repressor of temperate λ-phage (Japanese Published Unexamined Patent Application No.247389/1986). According to the method, it is required that the gene should preliminarily be integrated into the chromosomal DNA, by using temperate λ-phage, and then, genes present in the region of the chromosomal DNA into which the λ-phage DNA is to be integrated might possibly be damaged. However, it has not yet been known any method for efficiently producing an objective substance, by extrachromosomally regulating the intracellular retention and dissociation of a specific gene.

A method by means of a plasmid with a temperature-sensitive replication origin has been known as one of the methods for modifying chromosomal genes (Japanese Published Examined Patent Application No.108228/1995). The method comprises inserting, into a microbial cell, a recombinant DNA with an objective gene inserted into a plasmid with a temperature-sensitive replication origin, to thereby integrate the objective gene in the chromosomal DNA, and subsequently culturing the cell at a high temperature to dissociate the recombinant DNA from the cell, whereby the chromosomal integration of the gene or the dissociation of the gene on the chromosome can be carried out in a designated manner. More specifically, the primary purpose of the method resides in the permanent modification of a gene on a chromosome, and therefore, the method is not for regulating the extrachromosomal retention of a gene and dissociation thereof during culture, thereby attaining both microbial growth and the efficient production of an objective substance.

From the aforementioned respects, the present invention has been achieved. The object of the present invention is to provide a method for efficiently producing an objective substance, regulating the extrachromosomal retention of a gene with a disadvantageous action on the production of an objective substance, particularly with an advantageous action on the growth of a microorganism, thereby establishing both the growth of the microorganism and the production of the objective substance.

### Disclosure of Invention

The present inventors have made various investigations so as to attain the object, and consequently, the inventors have successfully regulated the intracellular retention of a specific gene and the dissociation thereof by using a plasmid containing a temperature-sensitive replication origin (referred to as "temperature-sensitive plasmid" hereinbelow), thereby attaining the efficient production of the objective substance. Thus, the present invention has been achieved. In other words, the present invention is a microorganism containing a plasmid carrying a gene with a disadvantageous action on the production of an objective substance and a temperature-sensitive replication origin.

Additionally, the present invention provides a method for producing an objective substance by a fermentation process, comprising a step of culturing and thereby proliferating the microorganism at a temperature at which the plasmid can replicate and a step of culturing the microorganism at a temperature at which the plasmid never can replicate to dissociate the plasmid from the cell to allow the microorganism to produce the objective substance.

As the objective substance, any substance that can be produced by a fermentation process using microorganisms and of which the production is disadvantageously affected with the action of a specific genetic product is satisfactory, with no specific limitation, and the objective substance specifically includes amino acids such as L-glutamic acid, L-lysine, L-phenylalanine, and tyrosine; nucleic acids such as guanylic acid and inosinic acid; vitamins; antibiotics; growth factors; biologically active substances; and protein. The present invention is applicable even to substances which are currently not produced by using microorganisms but can be biosynthesized in microorganisms.

The gene with a disadvantageous action on the production of an objective substance includes a gene requiring a specific substance for the production of the objective substance in the presence of the gene, in addition to a gene with an action to reduce the amount of the produced objective substance. More specifically, the gene includes genes encoding enzymes belonging to another pathway branching from the biosynthetic pathway of the objective substance, particularly a gene encoding an enzyme to reduce the amount of an intermediate flowing into the pathway. Additionally, the gene includes a gene decomposing the objective substance or a gene encoding an enzyme to decompose and convert the objective substance into other substances.

In accordance with the present invention, these genes preferably function advantageously for the growth of a microorganism. The gene advantageously functioning for the growth of a microorganism includes a gene bringing about better growth of the microorganism if the gene functions, compared with the case with no functioning thereof, and a gene never requiring a specific substance for the growth of the microorganism if the gene functions, which substance is essential for the growth of the microorganism if the gene does not function.

Specific combinations of the gene and the objective substance include for example a combination of dtsR gene or α-KGDH gene with L-glutamic acid, a combination of HD gene and L-lysine, a combination of tyrA gene and L-phenylalanine, a combination of pheA gene and L-tyrosine, a combination of purA gene and guanosine, a combination of guaB gene and adenosine, and a combination of purA gene and guaB gene with inosine.

The DTSR defective strain can produce L-glutamic acid at a distinctive level, under a condition of the presence of biotin at a level at which strains with the dtsR gene can hardly produce L-glutamic acid. Meanwhile, oleic acid is required to culture the DTSR defective stain.

α-KGDH defective strain can produce L-glutamic acid in the presence of an excess of biotin, with no addition of detergents or penicillin to the culture medium, while α-KGDH defective strain more poorly grows than the wild type strain.

Furthermore, HD defective strain does not synthetically produce L-threonine, so that L-lysine synthesis is progressed with no inhibition of aspartokinase activity which is under the influence of the feedback inhibition with L-threonine. Alternatively, because the HD defective strain cannot synthesize L-threonine or L-methionine, the HD defective strain cannot grow unless these amino acids are present in the culture medium.

The microorganism of the present invention retains a temperature sensitive plasmid carrying such a gene as described above and does not retain the gene being functional on the chromosomal DNA. Because the replication origin of the temperature-sensitive plasmid is temperature-sensitive, the plasmid can autonomously replicate at low temperature, for example 20 °C but never autonomously replicates at high temperature, for example 34 °C, and the copy number of the plasmid decreases following each division of the microorganism carrying the plasmid, so that the resulting microorganisms mostly do not retain the plasmid, leading to substantial dissociation of the gene. Therefore, transformants inserted with a temperature-sensitive plasmid carrying a specific gene can retain the gene intracellularly if cultured at low temperature; the transformants are defective in the gene if cultured at high temperature. As described above, the temperature-sensitive plasmid used in the present Examples is replicable at low temperature but is not replicable at high temperature, but if a temperature-sensitive replication origin not replicable at low temperature but replicable at high temperature is recovered, on the contrary, the replication origin may also be used. In the following description, the temperature at which the temperature-sensitive plasmid is never replicable is described as high temperature, with no intention to exclude the possibility that the temperature may be low temperature.

In order that the integration of the gene on the chromosome can be prevented during culturing at low temperature, which assures the dissociation of the gene, recA- strain is preferably used as the microorganism in accordance with the present invention,.

So as to enhance the effects on the retention and dissociation of the gene, the gene being functional is preferably retained only on the temperature-sensitive plasmid. More specifically, if the gene being functional is present on the chromosomal DNA or other plasmids other than the temperature-sensitive plasmid, the gene is retained intracellularly even after the temperature-sensitive plasmid is dissociated, so the effect owing to the plasmid dissociation is reduced. So as to allow the gene being functional to be retained only on the temperature-sensitive plasmid, for example, the gene present on the chromosome is subjected to mutation or damage, to block the production of the active products of the gene. Specifically, the promoter of the gene may satisfactorily be subjected to mutation, to block the expression of the gene; additionally, mutation including for example substitution, deletion, insertion, addition or rearrangement of bases may satisfactorily be induced in the coding region, to cause the expression products to lose their essential activities. Alternatively, the method for damaging the gene is for example a gene-damaging method by homologous recombination between deletion-type genes and the normal gene on the chromosome, and the method is preferable in view of the fact that the possibility of reverse mutation is extremely low. When a microorganism essentially never having any gene causing problems is used, the mutation or damage of the gene is not required.

The gene to be retained on the temperature-sensitive plasmid is not necessarily the same as the gene on the chromosomal DNA which is to be defective, and these genes may satisfactorily have substantially the same function. For example, an intrinsic gene present on the chromosome of a microorganism may be allowed to be defective, to make the microorganism retain a temperature-sensitive plasmid containing an exogenous gene having the same function as that of the intrinsic gene.

As the microorganism to be used in accordance with the present invention, any microorganism to which the genetic recombination technology is applicable and in which a temperature-sensitive replication origin is present may be usable, with no specific limitation, as long as the microorganism can be used for the fermentative production of an objective substance. Such microorganism includes for example coryneform bacteria, the bacteria of genus Escherichia, and the bacteria of genus Serratia. The present invention is also applicable to microorganisms currently not subjected to genetic recombination but possibly subjected to genetic recombination in future.

Specifically, the microorganism to be used in accordance with the present invention includes coryneform bacteria.

The coryneform bacteria referred to in accordance with the present invention mean a group of microorganisms defined by the Bergey's Manual of Determinative Bacteriology, the 8-th edition, page 599 (1974), which are bacillus, being aerobic, gram positive and non-acid-fast and not having a potency to form spore, including the bacteria of genus Brevibacterium, conventionally classified into genus Brevibacterium but currently integrated into the bacteria of genus Corynebacterium (Int. J. Syst. Bacteriol., 41, 255 (1981)), and additionally including the bacteria of genera Brevibacterium and Microbacterium, very close to the bacteria of genus Corynebacterium. Such coryneform bacteria include the following examples.
Corynebacterium acetoacidofilum
Corynebacterium acetoglutamicum
Corynebacterium callunae
Corynebacterium glutamicum
Corynebacterium lirium (Corynebacterium glutamicum)
Corynebacterium melacecola
Brevibacterium divaricatum (Corynebacterium glutamicum)
Brevibacterium flavum (Corynebacterium glutamicum)
Brevibacterium inmariofilum
Brevibacterium lactofermentum (Corynebacterium glutamicum)
Brevibacterium rhoseum
Brevibacterium saccharoliticum
Brevibacterium thiogenitalis
Brevibacterium thermoaminogenes

Specifically, bacterial strains as described below are illustrated.
Corynebacterium acetoacidofilum ATCC 13870
Corynebacterium acetoglutamicum ATCC 15806
Corynebacterium callunae ATCC 15991
Corynebacterium glutamicum ATCC 13020
Corynebacterium lirium (Corynebacterium glutamicum) ATCC 15990
Corynebacterium melacecola ATCC 17965
Brevibacterium divaricatum (Corynebacterium glutamicum) ATCC 14020
Brevibacterium flavum (Corynebacterium glutamicum) ATCC 14067
Brevibacterium inmariofilum ATCC 14068
Brevibacterium lactofermentum (Corynebacterium glutamicum) ATCC 13869
Brevibacterium rhoseum ATCC 13825
Brevibacterium saccharoliticum ATCC 14066
Brevibacterium thiogenitalis ATCC 19240
Corynebacterium thermoaminogenes AJ 12340 (FERM BP-1539)

These are available under supply from American Type Culture Collection (ATCC: 12301 Park Lawn Drive, Rockville, Maryland, 20852, USA) . Each microorganism has an accession number, and on the citation of the accession number, the microorganism can be supplied. The accession number of each microorganism is listed on the American Type Culture Collection Catalogue.

When the microorganism of the present invention is cultured at a temperature (low temperature) at which a temperature-sensitive plasmid is replicable, the plasmid is retained inside the cells of the microorganism, while the gene contained in the plasmid possibly functions. When the microorganism is cultured at a temperature (for example, high temperature) at which the temperature-sensitive plasmid cannot replicate, the gene is dissociated, together with the plasmid, from the cells. By culturing the microorganism of the present invention to a desired cell density at low temperature, thereafter culturing the microorganism at high temperature to dissociate the plasmid and further continuing the culture, accordingly, both the growth of the microorganism and the efficient production of the objective substance can be established together. Depending on the temperature preferable for the production of the objective substance after the dissociation of the plasmid, satisfactorily, the microorganism may be cultured as it is at high temperature, or the microorganism may be cultured at low temperature after the temperature is put back to the low temperature. If the microorganism is continued to be cultured at high temperature, the increase of the cells retaining the plasmid can be prevented. If the culturing at high temperature is not preferable for the production of the objective substance, the temperature should be put back to low temperature, to culture the microorganism. The remaining plasmid-retaining cells may sometimes increase if cultured at low temperature, but at the temperature, the production of the objective substance can be efficiently facilitated while cells with no plasmid retained therein are dominant.

In one embodiment of temperature shift, specifically, a method comprising carrying out seed culture at low temperature and carrying out culturing (primary culture) in a primary fermentative culture medium is illustrated. Furthermore, temperature shift may be conducted during the intermediate phase of preliminary culture or during the intermediate phase of primary culture. Still furthermore, the bacterial growth process and the plasmid dissociation process are not clearly discriminated from each other, and the plasmid dissociation process involves the bacterial growth.

The timing of temperature shift varies, depending on the gene, microorganism, temperature-sensitive replication origin and culture conditions to be used and the type of an objective substance, but the duration of culture at low temperature may readily be determined, by carrying out preliminary experiments at various culturing periods of time until temperature shift. Generally, a microorganism is cultured at the logarithmic growth phase until a desired cell density is reached, and thereafter, the temperature is shifted to a temperature at which the plasmid is never replicable.

Any culture medium is used for culture, with no specific limitation; culture media suitable for a microorganism to be used are satisfactorily used. For example, the culture medium for culturing coryneform bacteria is a common culture medium containing carbon sources, nitrogen sources, inorganic ions and other organic trace nutrients, if necessary.

As the carbon sources, use may be made of sugars such as glucose, lactose, galactose, fructose and starch hydrolysates; alcohols such as ethanol and inositol; organic acids such as acetic acid, fumaric acid, citric acid and succinic acid; and the like.

As the nitrogen sources, use may be made of inorganic ammonium salts such as ammonium sulfate, ammonium chloride, and ammonium phosphate; organic nitrogens such as soy bean hydrolysates; ammonia gas; ammonia water; and the like.

As the inorganic ions, small amounts of potassium phosphate, magnesium sulfate, iron ion, manganese ion and the like are supplemented. As the organic trace nutrients, appropriate amounts of essential substances such as vitamin B1 and yeast extract and the like may preferably be contained, if necessary.

Culturing is conducted under aerobic conditions for 16 to 72 hours; the culturing temperature is controlled to 20 to 45 ^{°}C; and the pH during culture is controlled to pH 5 to 8.5. For pH adjustment, furthermore, use may be made of acid or alkali substances, inorganic or organic, and ammonia gas and the like.

In accordance with the present invention, a strain with dtsR gene retained on a temperature-sensitive plasmid can produce L-glutamic acid at a prominent level, even in a culture medium containing an excess of biotin.

A strain with α-KGDH gene retained on a temperature-sensitive plasmid can produce L-glutamic acid even in a culture medium containing an excess of biotin, with no addition of detergents or penicillin to the culture medium. By adding detergents and penicillin to a culture medium or by regulating the biotin in a culture medium, the yield of glutamic acid may sometimes be further elevated.

A strain with HD gene retained on a temperature-sensitive plasmid can efficiently produce L-lysine even in a culture medium with no L-threonine or L-methionine contained therein.

The objective substance may satisfactorily be collected from a fermentative solution, in the same manner as in the method for producing substances by routine fermentative production. For example, amino acid can be collected by combinations of ion exchange resin process, precipitation process and other known processes.

### Brief Description of Drawings

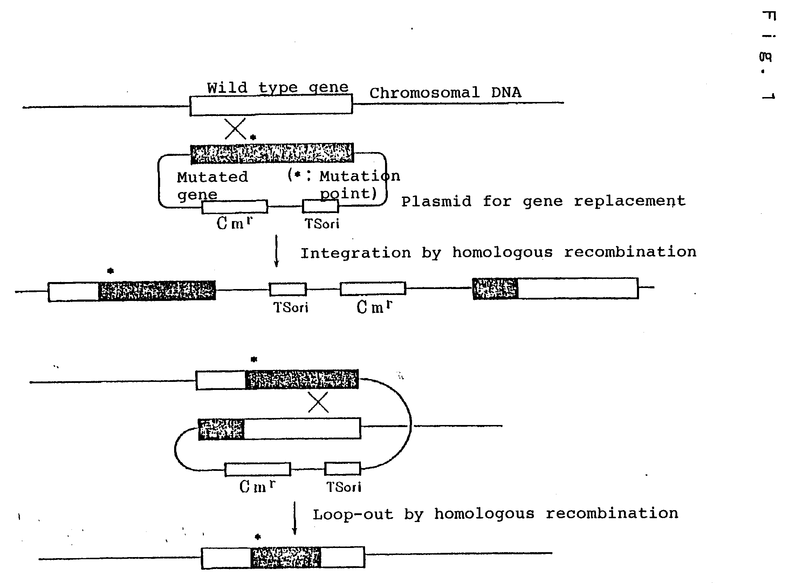

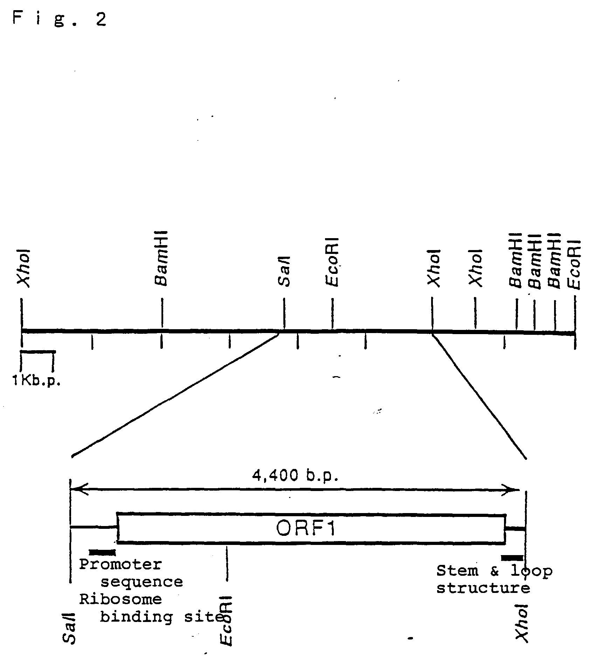

Fig.1 is a schematic view of gene integration and gene substitution: and Fig.2 is a restriction map of a DNA fragment containing α-KGDH gene.

### Best Mode for Carrying out the Invention

Description will be given hereinbelow about genes, microorganisms defective in the genes and temperature-sensitive plasmids, which are preferably used in accordance with the present invention. For specific description, herein, description about coryneform bacteria is primarily given, but the present invention is in no way limited to coryneform bacteria.

### 〈1〉 Genes to be used in accordance with the present invention

### (1) dtsR gene

dtsR gene is a gene isolated by the present inventors, which is a coryneform bacterium-derived gene responsible for detergent resistance. The nucleotide sequence of a DNA fragment containing the dtsR gene derived from a bacterium of genus Corynebacterium is denoted as SQ ID No.1. The coding region of the dtsR gene contains at least a sequence starting from ATG at positions 467 to 469 to CTG at positions 1985 to 1987 in the sequence.

An additional ATG (nucleotide Nos.359-361) is present in the same frame upstream the ATG at positions 467 to 469, and it cannot be denied the possibility that the ATG is the initiation codon, but based on the analysis of the sequence of the consensus sequence present in the upstream region of the gene, it is speculated that the ATG at positions 467 to 469 may be the initiation codon. More specifically, it is speculated that in the amino acid sequence denoted as SQ ID No.2, an amino acid sequence of amino acid Nos. 37 to 543 may be the amino acid sequence of DTSR protein. When the amino acid sequence of DTSR protein and the nucleotide sequence of dtsR gene are referred to in the present Specification, the ATG of Nos. 467 to 469 is sometimes described as the initiation codon, but it should additionally be noted the possibility that the ATG of Nos. 359 to 361 might be the initiation codon. When it is intended to insert the dtsR gene into a coryneform bacterium, it is supposed that the expression of the sequence of nucleotide Nos. 467 to 1987 in the nucleotide sequence No.1 may be satisfactorily required. A person skilled in the art will readily understand that the DTSR protein can be expressed accurately, if the coding region in the nucleotide sequence of SQ ID No.1 and an upstream region thereof, inclusive of the sequence of nucleotide Nos. 359 to 466, are inserted into a bacterium of genus Corynebacterium, irrespective of the fact that any one of the ATGs is the initiation codon. When the dtsR gene is expressed in the bacterium, the N-terminal Met encoded by the initiation codon may sometimes be cleaved with amino peptidase.

A transformant prepared by inserting plasmid pDTR6 containing the dtsR gene into Escherichia coli JM109 is designated AJ 12967 and deposited as Accession No. FERM P-14168 on February 22, 1994 at the Life Engineering and Industrial Technology Research Institute, the Agency of Industrial Science and Technology (1-3, Higashi 1-chome, Tsukuba-shi, Ibaraki 305, Japan), which is then transferred to the International Depository under the Budapest Treaty in February 9, 1995 and is given Accession No. FERM BP-4994 (the pamphlet of the International Publication WO 95/23224). DNA fragments containing the dtsR gene can be yielded, by recovering pDTR6 from the deposited bacterial strain and cleaving the plasmid DNA with restriction nucleases KpnI and XbaI.

The dtsR gene may also be yielded, by amplifying a DNA fragment containing the dtsR gene by polymerase chain reaction (PCR; see White, T. J. et al.; Trends Genet. 5, 185 (1989)] by using an oligonucleotide synthesized on the basis of the nucleotide sequence of SQ ID No.1 as primer and the chromosomal DNA of a coryneform bacterium as template. The primer to be used for PCR may satisfactorily have any sequence, provided that the primer can satisfy the following conditions:
nucleotide sequence is random at a (G + C) content around 50 %; the sequence does not form any specific secondary structure; and the strands are not complementary to each other. A primer of generally 20 to 30 nucleotides in length is frequently used. As such primer, additionally, preference is given to two oligonucleotide strands having complementary nucleotide sequences to the nucleotide sequences of both the 3' termini of the double-stranded DNA including at least the whole coding region of the dtsR gene.

According to conventional methods such as phosphoramidite process (Tetrahedron Letters, 22, 1859 (1981)) and by using commercially available DNA synthesizers (for example, DNA sequencer of Model 380B, manufactured by Applied Biosystems Inc.), oligonucleotides can be synthesized. PCR can be carried out, by using commercially available PCR systems (DNA Thermal Cycler Type PJ2000, manufactured by TaKaRa Brewery, etc.) and Taq DNA polymerase (supplied from TaKaRa Brewery), according to the processes instructed by the supplier.

By colony hybridization using oligonucleotides synthesized on the basis of the nucleotide sequence of SQ ID No.1 from the chromosomal DNA library of coryneform bacteria, DNA fragments containing the dtsR gene can be recovered.

Chromosomal DNA library can be prepared as follows. According to the method by Saito and Miura (H. Saito and K. Miura, Biochem. Biophys. Acta 72, 619 (1963)), chromosomal DNA is prepared from a coryneform bacterium. The chromosomal DNA is partially cleaved with restriction nucleases to recover a mixture of the resulting various fragments. Diverse types of restriction nucleases can be used, as long as the time of the cleavage reaction is controlled to regulate the extent of cleavage. For example, the chromosomal DNA is treated with Sau3AI at an enzyme unit of 1 to 10 units/ml at a temperature of 30 °C or more, preferably 37 °C, for various periods of time (1 minute to 2 hours), for digesting the DNA.

Then, the cleaved chromosomal DNA fragments are ligated to an autonomously replicable vector DNA in the cell of Escherichia coli, to prepare a recombinant DNA. More specifically, the vector DNA is treated with a restriction nuclease producing the same terminal nucleotide sequence as the sequence by the restriction nuclease Sau3AI used for the cleavage of the chromosomal DNA, for example BamHI, under conditions of an enzyme unit of 1 to 100 units/ml and a temperature of 30 °C or more for one hour or more, preferably 1 to 3 hours, for completely digesting the vector DNA, thereby cleaving the vector DNA. Then, the mixture of the chromosomal DNA fragments recovered as described above and the cleaved vector DNA are mixed together, and the resulting mixture is treated with DNA ligase, preferably T4 DNA ligase, under conditions of a temperature of 4 to 16 °C and an enzyme concentration of 1 to 100 units/ml for one hour or more, preferably 6 to 24 hours, to prepare recombinant DNAs.

The autonomously replicable vector in E. coli cells is preferably plasmid vector which is preferably autonomously replicable in a host, including for example pUC19, pUC18, pBR322, pHSG299, pHSG399, pHSG398 and RSF1010.

If DNA fragments with a potency to make plasmids autonomously replicable in coryneform bacteria [the DNA fragments can be prepared from for example pAM330 (see Japanese Published Unexamined Patent Application No.67699/1983), pHM1519 (see Japanese Published Unexamined Patent Application No.77895/1983), pCG1 (Japanese Published Unexamined Patent Application No.134500/1982), pCG2 (Japanese Published Unexamined Patent Application No.35197/1983), pCG4 (Japanese Published Unexamined Patent Application No.183799/1982), and pCG11 (Japanese Published Unexamined Patent Application No.183799/1982)], are inserted into these vectors, the resulting vectors can be used as so-called shuttle vectors autonomously replicable in both E. coli and coryneform bacteria.

Such shuttle vectors include what will be described hereinbelow. Furthermore, microorganisms carrying the individual vectors together with he Accession Nos. thereof at the International Depository Organization are shown in parenthesis.
- pAJ655: Escherichia coli AJ11882 (FERM BP-136)
Corynebacterium glutamicum SR8201 (ATCC 39135)
- pAJ1844: Escherichia coli AJ11883 (FERM BP-137)
Corynebacterium glutamicum SR8202 (ATCC 39136)
- pAJ611: Escherichia coli AJ11884 (FERM BP-138)
- pAJ3148: Corynebacterium glutamicum SR8203 (ATCC 39137)
- pAJ440: Bacillus subtilis AJ11901. (FERM BP-140)

By using the resulting recombinant DNAs, for example, E. coli strain K-12 is transformed, to prepare a chromosomal DNA library. The transformation can be executed according to the D. M. Morrison's method (Methods in Enzymology, 68, 326, 1979) or a method for increasing the DNA permeability comprising treating a receptor bacterial cell with calcium chloride [Mandel, M. and Higa, A., J. Mol. Biol., 53, 159 (1970)] and the like.

According to the method by P. Guerry et al. [J. Bacteriol., 116, 1064, (1973)] and the method by D. B. Clewell [J. Bacteriol., 110, 667, (1972)], for example, a recombinant DNA containing the dtsR gene can be isolated from transformants selected by hybridization.

Techniques for use in general genetic recombination, such as DNA cleavage and ligation, transformation, recombinant DNA extraction from transformants and colony hybridization, are described in detail in publications well known to a person skilled in the art, for example Molecular Cloning [Sambrook, J., Fritsch, E. F., Maniatis, T., Molecular Cloning, Cold Spring Harbor Laboratory Press (1989)].

### (2) HD gene

The HD gene of a coryneform bacterium has been recovered by the present inventors (the pamphlet of WO 95/23864). The nucleotide sequence of the HD gene of Brevibacterium lactofermentum strain AJ12036 (FERM BP-734) is shown as SQ ID No. 3 in the Sequence Listing, together with a putative amino acid sequence on the basis of the nucleotide sequence. Furthermore, the amino acid sequence is shown as SQ ID No.4 in the Sequence Listing. The comparison between the sequence and the sequence of the HD gene of Corynebacterium glutamicum reported by Peoples, et al.[Peoples, O. P. et al., Molecular Microbiology, 2(1), 63-72 (1988)] reveals that their nucleotides are different at four positions, and that the difference at one position among them is at amino acid level. Based on the sequence of the HD gene of Corynebacterium glutamicum, the difference is denoted as follows.
1. 531G → C (148Gly → 148Ala)
2. 1222G → C
3. 1318G → T
4. 1324C → G

Such difference between the sequences of the HD genes of individual wild type strains of coryneform bacteria does not have any influence on the HD activity, and therefore, the sequence of the HD gene of Corynebacterium glutamicum and the sequence of the HD gene of Brevibacterium lactofermentum can be handled identically. In the HD genes, furthermore, the methionine residue encoded by an initiation codon may possibly be removed.

The HD gene used in the present Examples is recovered, by amplifying a DNA fragment containing the HD gene according to PCR using as primers oligonucleotides (SQ ID Nos. 5 and 6) synthetically prepared on the basis of the known sequence of Corynebacterium glutamicum [Peoples, O. P. et al.; Molecular Microbiology, 2(1), 63-72 (1983)] and as template the chromosomal DNA of Brevibacterium lactofermentum strain AJ12036 (FERM BP-734).

Primers to be used by the PCR are not limited to those described above and can be prepared on the basis of the nucleotide sequence denoted as SQ ID No. 3. Primers may have any sequence, provided that the nucleotide composition is random at a (G + C) content around 50 %; that the sequence does not construct any specific secondary structure; and that the strands are not complementary to each other. Generally, primers of a length of 20 to 30 nucleotides are frequently used. The primers are preferably two oligonucleotide strands, having complementary nucleotide sequences to the nucleotide sequences at both the 3' termini of a double-stranded DNA containing at least the whole coding region of the HD gene.

By hybridization using as probe an oligonucleotide synthetically produced on the basis of the nucleotide sequence denoted as SQ ID No.3, a DNA fragment containing the HD gene can be recovered from the chromosomal DNA library of the coryneform bacterium.

If the chromosomal DNA library is prepared by using E. coli, a sequence to be used in a probe is preferably selected from the region corresponding to about 100 amino acid residues at the C terminus of the HD. The reason is as follows: because it has been known that E. coli has two types of HD gene (HD-1, HD-2) (Zakin, M. M. et al; J. R. C., 258, 3028-3031 (1983)) and the region corresponding to about 100 amino acid residues at the C terminus of Corynebacterium glutamicum HD is present in neither of them, a sequence to be used in a probe never hybridizes with the HD gene on the chromosome of E. coli, if selected from the region.

If an amplified DNA fragment does not contain the whole length of the gene encoding HD, the chromosomal DNA is cut with a restriction nuclease different from the restriction nuclease used for the preparation of the chromosomal DNA library, to prepare again a chromosomal DNA library, for carrying out again the selection by hybridization and the analysis of restriction fragments, to recover a DNA fragment containing the whole length of the HD gene. By using the DNA fragment first recovered as the probe, then, hybridization can readily be facilitated.

Oligonucleotide synthesis and the preparation of chromosomal DNA library can be conducted in the same manner as described in (1).

### (3) α-KGDH gene

The α-KGDH gene of a coryneform bacterium has been recovered by the present inventors (the pamphlet of the International Publication WO 95/34 672). The nucleotide sequence of a DNA fragment containing the α-KGDH gene derived from Brevibacterium lactofermentum ATCC13869 is shown as SQ ID No. 7 in the Sequence Listing. Based on the nucleotide sequence, an open reading frame is speculated, and an amino acid sequence of a product speculated on the basis of the nucleotide sequence is denoted as SQ ID No. 8. It is well known that because the methionine residue at the N terminus of a protein is derived from ATG as initiation codon, the methionine residue has no relation with the essential functions of the protein, in most cases, so the residue is removed by the action of peptidase after translation. In the case of the α-KGDH protein, the removal of the methionine residue may possibly occur.

A transformant recovered by inserting plasmid pPKS-X containing α-KGDH gene into Brevibacterium lactofermentum AJ11060 (Japanese Published Examined Patent Application No.10797/1984) is designated as Brevibacterium lactofermentum AJ12999 and is deposited as Accession No. FERM P-14349 on date June 3, 1994 at the Life Engineering and Industrial Technology Research Institute, the Agency of Industrial Science and Technology (1-3, Higashi 1-chome, Tsukuba-shi, Ibaraki 305, Japan), which is then transferred to the International Depository under the Budapest Treaty on date June 2, 1995, given Accession No. FERM BP-5123. DNA fragments containing the α -KGDH gene can be yielded, by recovering pPKS-X from the deposited bacterial strain and cleaving the plasmid DNA with restriction nucleases SalI and XbaI.

It has been known that the α-KGDH complex of Escherichia coli is composed of three subunits, namely E1 (α-ketoglutarate dehydrogenase: EC 1.2.4.2.), E2 (dihydrolipoamide succinyl transferase: EC 2.3.1.61.) and E3 (lipoamide dehydrogenase: 1.6.4.3.) and E1 and E2 genes form an operon structure, while pyruvate dehydrogenase ( EC 1.2.4.1.) has also the E3 in common. The nucleotide sequences of the E1 and E2 genes of Escherichia coli have been elucidated (Eur. J. Biochem., 141, 351 (1984), Eur. J. Biochem., 141, 361 (1984)).

Additionally, the nucleotide sequences of the E1 and E2 genes of Bacillus subtilis have been elucidated as well (J. Bacteriol., 171, 3667 (1989), Gene, 61, 217 (1987), etc.).

The α-KGDH gene derived from Brevibacterium lactofermentum ATCC 13869 is isolated and cloned, by utilizing the homology in the nucleotide sequence of E1 gene between Escherichia coli and Bacillus subtilis. More specifically, a DNA fragment containing α-KGDH gene was amplified, by selecting a region at a high homology in α-KGDH E1 subunit between Escherichia coli and Bacillus subtilis and according to PCR using the synthetic oligonucleotides denoted as SQ ID Nos. 9 and 10 as primers and the chromosomal DNA of Brevibacterium lactofermentum strain ATCC13869 as template.

The primers to be used for PCR are not limited to those described above, but the primers can be prepared on the basis of the nucleotide sequence of SQ ID No. 7. Any primer may be satisfactory, as long as the primer can satisfy the provisions that the nucleotide composition is random at a (G + C) content around 50 %; that the sequence does not form any specific secondary structure; and that the strands are not complementary to each other. A primer of generally 20 to 30 nucleotides in length is frequently used. As such primer, additionally, preference is given to two oligonucleotide strands with complementary nucleotide sequences to the nucleotide sequences of both the 3' termini of the double-stranded DNA including at least the whole coding region of the α-KGDH gene.

According to hybridization using as primer an oligonucleotide synthetically produced on the basis of the nucleotide sequence denoted as SQ ID No. 7, a DNA fragment containing α-KGDH gene can be recovered from the chromosomal DNA library of a coryneform bacterium.

The synthesis of the oligonucleotide and the preparation of the chromosomal DNA library can be performed in the same manner as described in (1).

### 〈2〉 A plasmid carrying a temperature-sensitive replication origin

Temperature-sensitive replication origin can be yielded by subjecting a chemically resistant plasmid autonomously replicable in the cells of a microorganism to mutation treatment and transforming the microorganism with the plasmid and recovering a plasmid from a transformant strain capable of growing in a culture medium containing a chemical agent at low temperature but never growing therein at high temperature.

The mutation treatment of plasmids includes for example a method comprising treating plasmids in vitro with hydroxylamine (G. O. Humpherys et al: Molec. Gen. Genet. 145, 101-108 (1976)).

The term "low temperature" herein referred to is a concept relative to "high temperature". The border between low temperature and high temperature is not specifically limited, but in the narrowest sense, the term "low temperature" means a temperature zone in which microorganisms can grow when cultured and the term "high temperature" means a temperature zone in which microorganisms of themselves are not killed. The border between low temperature and high temperature can be determined by culturing a transformant carrying a temperature-sensitive plasmid in a culture medium containing chemical agents at various temperatures and evaluating the lower limit of the temperature (zone) in which the plasmid cannot grow.

Plasmids functioning in the cells of coryneform bacteria and having a temperature-sensitive replication origin include pHS4, pHS22, and pHS23. These are autonomously replicable in both the bacteria of Escherichia coli and coryneform bacteria, which are plasmids recovered from a transformant capable of growing on an M-CM2G plate containing 25 µg/ml kanamycin at low temperature (20 °C ) but never growing at high temperature (34 °C), which transformant is prepared by subjecting a kanamycin-resistant plasmid vector pHK4 to hydroxylamine treatment, and transforming Brevibacterium lactofermentum (Japanese Published Examined Patent Application No.108228/1995). Escherichia coli AJ12570 carrying pHS4 is deposited as Accession No. FERM P-11762 on date October 11, 1990 at the Life Engineering and Industrial Technology Research Institute, the Agency of Industrial Science and Technology (1-3, Higashi 1-chome, Tsukuba-shi, Ibaraki 305, Japan), which is then transferred to the International Depository under the Budapest Treaty on date August 26, 1991, given Accession No. FERM BP-3523.

Plasmids pHSC4, pHSC22 and pHSC23 recovered by conjugating individual DNA fragments containing the coryneform bacteria-derived replication origins cleaved out from pHS4, pHS22 and pHS23 to pHSG398 as vectors for Escherichia coli can also be used as temperature-sensitive plasmids. pHSC4, pHSC22 and pHSC23 autonomously grow in coryneform bacteria and Escherichia coli, to impart chloramphenicol resistance to the hosts (Japanese Published Examined Patent Application No.108228/1995). Escherichia coli AJ12571 carrying pHSC4 is deposited as Accession No. FERM P-11763 on date October 11, 1990 at the Life Engineering and Industrial Technology Research Institute, the Agency of Industrial Science and Technology (1-3, Higashi 1-chome, Tsukuba-shi, Ibaraki 305, Japan), which is then transferred to the International Depository under the Budapest Treaty on date August 26, 1991, given Accession No. FERM BP-3524. Escherichia coli AJ12615 carrying pHSC22 and Escherichia coli AJ12616 carrying pHSC23 are deposited as Accession Nos. FERM P-12213 and FERM 2-12214, respectively, on date April 24, 1991 at the Life Engineering and Industrial Technology Research Institute, the Agency of Industrial Science and Technology (1-3, Higashi 1-chome, Tsukuba-shi, Ibaraki 305, Japan), which is then transferred to the International Depository under the Budapest Treaty on date August 26, 1991, given Accession Nos. FERM BP-3530 and FERM BP-3531, respectively.

These temperature-sensitive plasmids can autonomously grow in the cells of coryneform bacteria at about 10 to 32 °C, but never can grow at about 34 °C or more.

DNA fragments having temperature-sensitive replication origins can be recovered, by cleaving out the pHSC4 with BamHI and KpnI.

The nucleotide sequences of the regions containing the temperature-sensitive replication origins in the individual plasmids are described in the publication of Japanese Published Examined Patent Application No.108228/1995.

The temperature-sensitive plasmid to be used in accordance with the present invention contains a temperature-sensitive replication origin and a gene disadvantageously functioning for the production of an objective substance. Such plasmid can be recovered, by ligating a DNA fragment containing a temperature-sensitive replication origin to a DNA fragment containing the gene with ligases such as T4 DNA ligase. Additionally, a temperature-sensitive plasmid containing an origin of replication functioning in E. coli and markers such as chemically resistant gene in addition to these DNA fragments is convenient for the preparation of plasmids.

### 〈3〉 Microorganisms for use as hosts

### (1) Gene-defective strain

The microorganism of the present invention is a microorganism wherein a gene disadvantageously serving for the production of an objective substance as described above is preferably present only on the plasmid. Such microorganism can be recovered, by transforming a host microorganism never carrying a functional gene on the chromosomal DNA with a temperature-sensitive plasmid containing the gene. The host microorganism never carrying the gene never functioning on the chromosomal DNA can be recovered by subjecting individual genes present on the chromosome to mutation so as to block the normal functioning. The mutation may be a type of mutation to suppress the transcription or translation of a gene or may be a type of mutation to deplete a part or the whole of a gene, namely to destroy the gene. A strain never carrying a gene being functional is hereinbelow referred to as "defective strain".

Gene-defective strain can also be recovered, by subjecting a microorganism producing a wild-type gene to ultraviolet irradiation or chemical treatment and selecting a strain never producing the genetic products substantially having a function. Furthermore, a gene-defective strain can be recovered by a culturing process by genetic recombination. When a gene is recovered, in particular, the destruction of the gene is readily performed by homologous recombination process using genetic recombination process. The gene destruction by homologous recombination has already been established, and a process using linear DNAs and a process using temperature-sensitive plasmids are applicable.

More specifically, the substitution, deletion, insertion, addition or reverse orientation of one or plural nucleotides is allowed to occur in the coding region or promoter region of a gene intended to be defective, by site- specific mutation process (Kramer, W. and Frits, H. J., Methods in Enzymology, 154, 350 (1987)) and chemical treatments with sodium hyposulfite and hydroxylamine (Shortle, D. and Nathans, D., Proc. Natl. Acad. Sci. USA, 75, 270 (1978)), and the gene modified or destroyed in such manner is substituted with the normal gene on the chromosome, whereby the activity of the gene product can be reduced or lost or the transcription of the gene can be reduced or abolished.

The site-specific mutation process is a process using synthetic oligonucleotides, by which an arbitrary substitution, deletion, insertion, addition or reverse orientation can be introduced into only one arbitrary limited base pair. So as to utilize the process, a plasmid having an objective gene having been cloned and having the DNA nucleotide sequence determined is first modified to prepare single strands. Then, a synthetic oligonucleotide complementary to a region intended to be mutated is to be synthetically prepared, and the resulting synthetic oligonucleotide does not have an absolutely complementary sequence, but then, the oligonucleotide should have the substitution, deletion, insertion, addition or reverse orientation of an arbitrary nucleotide. Thereafter, the single-stranded DNA is annealed with the synthetic oligonucleotide having the substitution, deletion, insertion, addition or reverse orientation of an arbitrary nucleotide, to synthesize then a complete double-stranded plasmid by using the Klenow fragment of DNA polymerase I and T4 ligase, which is introduced into the competent cell of Escherichia coli. Some of the resulting transformants thus recovered have plasmids containing genes with an arbitrary substitution, deletion, insertion, addition or reverse orientation fixed therein. Similar methods capable of inserting, modifying or destroying gene mutation include recombinant PCR (PCR Technology, Stockton Press (1989)).

Additionally, a method using chemical treatment is a method for randomly introducing mutation having nucleotide substitution, deletion, insertion, addition or reverse orientation into a DNA fragment, by directly treating the DNA fragment containing an objective gene with sodium hyposulfite, hydroxylamine and the like.

The method for substituting the gene thus modified or destroyed by introducing the thus recovered mutation with the normal gene on the chromosome of a microorganism such as coryneform bacteria, includes a method utilizing homologous recombination (Experiments in Molecular Genetics, Cold Spring Harbor Laboratory Press (1972); Matsuyama, S. and Mizushima, S., J. Bacteriol., 162, 1196 (1985)). Homologous recombination proceeds as follows. When a plasmid or the like having a sequence with homology to the sequence on a chromosome is introduced into bacteria, recombination occurs at a position with homology at a certain frequency, so that the introduced plasmid is wholly integrated on the chromosome. When recombination occurs subsequently at the position with homology on the chromosome, the plasmid is again dissociated from the chromosome. Depending on the position where recombination occurs, in some case, the mutation-introduced gene may then rather be fixed on the chromosome while the intact normal gene may be dissociated along with the plasmid from the chromosome, in some case. By selecting such a bacterial strain, the gene modified or destroyed by introducing mutation with nucleotide substitution, deletion, insertion, addition or reverse orientation therein is substituted with the normal gene on the chromosome in the bacterial strain, which is then recovered.

The method for destroying gene by homologous recombination will now be specifically described in an example of a coryneform bacterial strain with the α-KGDH gene destroyed therein (Fig.1)

A transformant strain with a recombinant DNA integrated into the chromosomal DNA therein can be recovered, by inserting a temperature-sensitive replication origin derived from Brevibacterium lactofermentum, a mutant α-KGDH gene and a marker gene exerting resistance to a drug such as chloramphenicol into a plasmid vector to prepare a recombinant DNA, transforming a coryneform bacterium with the recombinant DNA, culturing the resulting transformant strain at a temperature at which the temperature-sensitive replication origin does not function, and continuously culturing the transformant strain in a culture medium containing the drug.

In the strain with the recombinant DNA thus integrated in the chromosome, recombination with the α-KGDH gene sequence essentially present on the chromosome occurs, so that two fusion genes composed of the chromosomal α-KGDH gene and the mutant α-KGDH gene are inserted in the chromosome while the fusion genes hold the remaining part (the vector part, the temperature-sensitive replication origin and the chemical-resistant marker) of the recombinant DNA between them. Because the wild-type α-KGDH is therefore dominant at that state, the strain exerts the same growth level as the growth level of the wild type strain.

So as to leave only the mutant α-KGDH gene on the chromosomal DNA, subsequently, one copy of the α-KGDH gene is dissociated together with the vector part (including the temperature-sensitive replication origin and the chemical-resistant marker) through the recombination of the two α-KGDH genes. For example, the chromosome-integrated strain is cultured and is then inoculated on a plate culture medium containing a drug, for culturing. The grown colonies are subjected to replica culture on a plate culture medium containing a drug, to recover a drug-susceptible strain. The dissociation of the vector part from the chromosome of the resulting drug-susceptible stain is confirmed by Southern hybridization, while no expression of normal α-KGDH is also confirmed.

If gene substitution is conducted by using, as the mutant α-KGDH gene, an α-KGDH gene encoding a part of the α-KGDH, namely a partially defective α-KGDH gene, an α-KGDH gene-destroyed strain can be recovered, in which the chromosomal α-KGDH gene is preliminarily substituted with the partially defective α-KGDH gene.

In the same manner as described above, a dtsR gene-defective strain and an HD gene-defective strain can be recovered. For preparing an HD gene-destroyed strain, the part to be defective in the HD gene is an N-terminal region, for example, a region starting from the N terminus up to the 350-th amino acid, for example a region of amino acids 100-200, or a region of amino acids 250-350 because it is speculated that the N-terminal region may be responsible for the activity. Because the HD gene and the homoserine kinase present downstream the gene are within the same operon, preferably, the promoter site of the HD gene should not be defective in order not to inhibit the expression of the homoserine kinase.

The integration of a recombinant DNA inside the cells of coryneform bacteria can be done by a process of increasing the DNA permeability by treating a receptor bacterial cell with calcium chloride as reported about E. coli K-12 (Mandel, M. and Higa, A., J. Mol. Biol., 53, 159 (1970)), or a process of integrating the recombinant DNA into a cell at a growth phase (so-called competent cell) so that the cell might incorporate the DNA as reported about Bacillus subtilis (Duncan, C. H., Wilson, G. A. and Young, F. E., Gene, 1, 153 (1977)). Otherwise, as known about Bacillus subtilis, actinomycetes and yeast (Chang, S. and Choen, S. N., Molec. Gen. Genet., 168.111 (1979); Bibb, M. J., Ward, J. M. and Hopwood, O. A., Nature, 274, 398 (1978); Hinnen, A., Hicks, J. B. and Fink, G. R., Proc. Natl. Acad. Sci. USA, 75, 1929 (1978)), a DNA receptor bacterium is prepared as a recombinant DNA receptor bacterium, by preparing the DNA receptor bacterium as a protoplast or spheroplast which can readily integrate a recombinant DNA.

Among protoplast processes, the aforementioned process for use in Bacillus subtilis can achieve a satisfactorily high frequency, or a process of inserting DNA into the protoplast of genus Corynebacterium or Brevibacterium in the presence of polyethylene glycol or polyvinyl alcohol with a divalent metal ion as disclosed in Japanese Published Unexamined Patent Application No. 183799/1982 is also utilized, with no doubt. The same results can be brought about by a process of promoting the integration of DNA by adding carboxymethyl cellulose, dextran, Ficoll, and Pluronic F68 (Cerva Co.) instead of polyethylene glycol or polyvinyl alcohol.

By an electric pulse process (Sugimoto et al., Japanese Published Unexamined Patent Application No.207791/1991), furthermore, a recombinant DNA can be integrated into a receptor bacterium belonging to genus Brevibacterium or Corynebacterium.

The HD-destroyed strain derived from Brevibacterium lactofermentum strain AJ12036 (FERM BP-734), for example, as one of gene-destroyed strains for use in accordance with the present invention, is designated Brevibacterium lactofermentum AJ12846, and is then deposited as Accession No. FERM P-14197 on date March 1, 1994 at the Life Engineering and Industrial Technology Research Institute, the Agency of Industrial Science Technology (1-3, Higashi 1-chome, Tsukuba-shi, Ibaraki 305, Japan), which is then transferred to the International Depository under the Budapest Treaty on date February 9, 1995, given Accession No. FERM BP-4995.

Host microorganisms into which it is intended to insert the temperature-sensitive plasmid are preferably recA- mutant strains. When a strain carrying the temperature-sensitive plasmid is cultured at low temperature, homologous recombination between the normal gene contained in the plasmid and the mutant gene on the chromosomal DNA occurs, involving the possibility of the integration of the normal gene into the chromosome. Once the integration occurs, the strain is cultured at high temperature, so the normal gene of interest remains along with the plasmid DNA on the chromosome, even after the plasmid is allowed to be dissociated from the host cell. So as to block such homologous recombination, preferably, recA- mutant strains are used.

The recA gene of coryneform bacteria has already been isolated, and the nucleotide sequence is also known (Japanese Published Unexamined Patent Application No.322879/1995), which is registered on a database (EMBL Accession No. X77384). Based on the sequence, a primer is prepared, whereby the recA gene can be isolated by PCR process. By using the resulting recA gene to recover a recA gene-destroyed strain in the same manner as described above, a recA- strain can be recovered. It is supported by examining the sensitivity to DNA-damaging chemical agents such as mitomycin C and methyl methanesulfonate or to UV irradiation, or by examining the homologous recombination potency that the resulting strain is a recA- strain. Compared with recA+ strain, strain recA- has increased sensitivity to the chemical agents and to UV irradiation and decreased homologous recombination potency. The creation of a recA gene-destroyed strain by using the inner sequence of the recA gene after amplification by PCR process is described in detail in R. Fritzpatrick et al., Appl. Microbiol. Biotechnol. (1994) 42, 575-580.

When recA+ strain is used as a host, cells with the gene of interest being inserted into the chromosomal DNA may emerge. As long as cells with no occurrence of integration of the plasmid DNA therein occupy a major ratio during culture, the emergence of such cells is not problematic. In such case, cells with no occurrence of the integration of the gene of interest in the chromosomal DNA are subjected to single colony isolation, satisfactorily, for use in producing an objective substance. When not any region homologous to the gene disadvantageously serving for the production of an objective substance is present on the chromosomal DNA of a host microorganism, the aforementioned problem relating to insertion does not occur even if recA+ strain is used.

### (2) Microorganism suitable for the production of an objective substance

As the microorganism for use for recovering a gene-defective strain, a microorganism with a potency to produce an objective substance should be used. As specific examples of the microorganism producing an objective substance, various L-amino acid producing bacteria of coryneform bacteria are described below.

### (i) L-lysine producing bacteria

Various artificial mutant strains have been known conventionally as the L-lysine producing bacteria of coryneform bacteria. Such artificial mutant strains include what will be described below:
S-(2-aminoethyl)-cysteine (abbreviated as "AEC" hereinafter)-resistant mutant strain;
mutant strain requiring amino acids such as L-homoserine for the growth thereof (Japanese Published Examined Patent Application Nos. 28078/1973 and 6499/1981);
mutant strain resistant to AEC and essentially requiring amino acids such as example L-leuclne, L-homoserine, L-proline, L-serine, L-arginine, L-alanine and L-valine (USP Nos. 3708395 and 3825472);
L-lysine-producing mutant strain resistant to DL-α-amino-ε caprolactam, α-amino-lauryl lactase, aspartate analogs, sulfa agents, quinoide, and N-lauroyl leucine;
L-lysine-producing mutant strain resistant to oxalacetate dehydrogenase or inhibitors of respiratory enzymes (Japanese Published Unexamined Patent Application Nos. 53588/1975, 31093/1975, 102498/1977, 9394/1978, 86089/1978, 9793/1980, 9759/1980, 32995/1981 and 39778/1981; Japanese Published Examined Patent Application Nos. 43591/1978 and 1833/1978);
L-lysine-producing mutant strain requiring inositol or acetic acid (Japanese Published Unexamined Patent Application Nos. 9784/1980 and 8692/1981);
L-lysine-producing mutant strain with sensitivity to fluoropyruvic acid or a temperature of 34 °C or more (Japanese Published Unexamined Patent Application Nos. 9783/1980 and 86090/1978); and
L-lysine-producing mutant strain resistant to ethylene glycol, from Brevibacterium or Corynebacterium (USP No.4411997) et al.

More specifically, the following strains are listed.
Brevibacterium lactofermentum AJ12031 (FERM BP-277, Japanese Published Unexamined Patent Application No.62994/1985)
Brevibacterium lactofermentum ATCC 39134 (Japanese Published Unexamined Patent Application No.62994/1985)
Corynebacterium glutamicum AJ3463 (FERM P-1987, Japanese Published Examined Patent Application No. 34477/1976)
Brevibacterium lactofermentum AJ12435 (FERM BP-2294, USP No. 5,304,476)
Brevibacterium lactofermentum AJ12592 (FERM BP-3239, USP No. 5,304,476)
Corynebacterium glutamicum AJ12596 (FERM BP-3242, USP No. 5,304,476)
Brevibacterium lactofermentum AJ11446 (FERM P-5163, Japanese Published Unexamined Patent Application No. 207791/1990)

As E. coli producing L-lysine, for example, a mutant strain with resistance to an L-lysine analog is illustrated. The L-lysine analog inhibits the growth of bacteria of genus Escherichia, but the inhibition is wholly or partially released, only if L-lysine is concurrently present in the culture medium. For example, the L-lysine analog includes oxazolidine, lysine hydroxamate, AEC, γ-methyl lysine, and α-chlorocaprolactam. Mutant strains resistant to these lysine analogs can be prepared, by subjecting microorganisms of genus Escherichia to general artificial mutation manipulations. The bacterial strain for use in preparing L-lysine specifically includes Escherichia coli AJ11442 (FERM BP-1543, NRRL B-12185; see Japanese Published Unexamined Patent Application No. 18596/1981 and USP No. 4,346,170). The strain AJ11442 is deposited as Accession No. FERM P-5084 on date May 1, 1981 at the Life Engineering and Industrial Technology Research Institute, the Agency of Industrial Science and Technology (1-3, Higashi 1-chome, Tsukuba-shi, Ibaraki 305, Japan), which is then transferred from the original depository to the International Depository under the Budapest Treaty on date October 29, 1987, given Accession No. FERM BP-1543. In these microorganisms, the aspartokinase is released from the feedback inhibition by L-lysine.

In addition, for example, L-threonine-producing bacteria are listed. The reason is that the inhibition of aspartokinase by L-lysine is generally released in the L-threonine-producing bacteria. Among currently known L-threonine-producing bacteria of E. coli, VKPM B-3966 strain has the highest production potency. The VKPM B-3966 strain is deposited under Registration No. RIA 1867 on date November 19, 1987 at USSR All-Union Scientific Center of Antibiotics (Nagatinskaya Street 3-A, 113105 Moscow; Russian Federation).

In the aforementioned L-lysine-producing bacteria, for example, the amplification of the gene for L-lysine biosynthesis may be enhanced. Such gene includes for example a gene encoding phosphoenol pyruvate carboxylase having a mutation to release the feedback inhibition by aspartic acid (see Japanese Published Examined Patent Application No. 83714/1995).

### (ii) L-glutamate-producing bacteria

Coryneform bacteria having the potency to produce L-glutamic acid include wild type strains of coryneform bacteria and with a potency to produce glutamic acid or mutant strains derived therefrom. Such mutant strains include for example what will be described below:
Brevibacterium lactofermentum AJ12475 (FERM BP-2922, USP No. 5,272,067);
Brevibacterium lactofermentum AJ12476 (FERM BP-2923, USP No. 5,272,067);
Brevibacterium flavum AJ12477 (FERM BP-2924, USP No. 5,272,067);
Corynebacterium glutamicum AJ12478 (FERM BP-2925, USP No. 5,272,067); and
Corynebacterium glutamicum ATCC 21492.

The L-glutamate-producing bacteria of Escherichia coli include for example Escherichia coli AJ12628 (FERM BP-3854), Escherichia coli AJ12624 (FERM BP-3853; see France Patent Published Application No. 2,680, 178), and Escherichia coli AJ12949 (FERM BP-4881; see European Patent Published Application No. 0,670,370).

So as to improve the potency to produce L-glutamic acid, the gene for L-glutamate biosynthesis in the glutamic acid-producing bacteria may satisfactorily be enhanced. Examples of the enhancement of the gene for the glutamate biosynthesis reside in phosphofructokinase in glycolytic pathway (PFK, Japanese Published Unexamined Patent Application No. 102692/1988), phosphoenol pyruvate carboxylase in anaplerotic pathway (PEPC, Japanese Published Examined Patent Application No. 83714/1995, Japanese Published Unexamined Patent Application No. 55089/1987), citrate synthase in TCA cycle (CS, Japanese Published Unexamined Patent Application No. 201582/1987, Japanese Published Unexamined Patent Application No. 119688/1988), aconitic acid hydrolase (ACO, Japanese Published Unexamined Patent Application No. 294086/1987), isocitrate dehydrogenase (ICDH, Japanese Published Unexamined Patent Application Nos. 166890/1987 and 214189/1988), and glutamate dehydrogenase (GDH, Japanese Published Unexamined Patent Application No. 268185/1986) involved in amination.

### (iii) L-phenylalanine-producing bacteria

The L-phenylalanine-producing bacteria of coryneform bacteria include Brevibacterium lactofermentum AJ12637 (FERM BP-4160) (see France Patent Published Application No. 2,686,898): the L-phenylalanine-producing bacteria of Escherichia coli include Escherichia coli AJ12604 (FERM BP-3579) (see European Patent Published Application No. 488,424). The strain AJ12604 is a transformant strain prepared by independently inserting a plasmid containing a mutant aroG gene and a plasmid containing a mutant pheA gene into a host tyrA- E. coli (see Japanese Published Unexamined Patent Application No. 344811/1993). The strain AJ12604 is deposited as Accession No. FERM P-11975 on date January 28, 1991 at the Life Engineering and Industrial Technology Research Institute, the Agency of Industrial Science and Technology (1-3, Higashi 1-chome, Tsukuba-shi, Ibaraki 305, Japan), which is then transferred from the original depository to the International Depository under the Budapest Treaty on date September 26, 1991, given Accession No. FERM BP-3579.

### Examples

The present invention will more specifically be described hereinbelow, while showing the examples of the present invention, along with the preparation examples of genes and host microorganisms preferable for use in accordance with the present invention.

### (Example 1)

### Creation of an L-glutamate-producing bacterium by utilizing dtsR gene and production of L-glutamic acid by using the bacterium

### 〈1〉 Preparation of temperature-sensitive plasmid containing dtsR gene

The plasmid was recovered from Escherichia coli JM109/pDTR6 (Private Number AJ12967; Accession No. FERM BP-4994) carrying plasmid pDTR6 (the pamphlet of the International Publication WO 95/34672) containing the dtsR gene derived from Brevibacterium lactofermentum strain ATCC 13869. Herein, the pDTR6 is a recombinant plasmid with a vector plasmid pSAC4 autonomously replicable in bacteria of both Escherichia coli and genus Corynebacterium, and has a chloramphenicol-resistant marker. Additionally, a transformant strain prepared by inserting pDTR6 into a coryneform bacterial mutant strain with increased sensitivity to detergents, namely a Brevibacterium lactofermentum strain AJ11060, had increased resistance to a detergent (polyoxyethylene sorbitan monopalmitate).

pDTR6 was cleaved with restriction nucleases KpnI and XbaI, to recover a DNA fragment containing the dtsR gene. By using T4 DNA ligase, the resulting DNA fragment was ligated to plasmid pHSG398 (manufactured by TaKaRa Brewery) after cleavage with KpnI and XbaI, to prepare pHSGX-K. pHSGX-K is replicable autonomously in the bacteria of Escherichia coli.

At the KpnI site of the pHSGX-K thus recovered was inserted a temperature-sensitive replication origin (referred to as "TSori" hereinafter) derived from a coryneform bacterium. A DNA fragment containing TSori was recovered by cleaving plasmid pHSC4 containing TSori with BamHI and KpnI blunt ending both the termini of the resulting DNA fragment by using a blunting kit manufactured by TaKaRa Brewery for subsequent conjugation with a KpnI linker (manufactured by TaKaRa Brewery), thereafter subjecting the resulting fragment to self-ligation to prepare plasmid pKCT4, and cutting the plasmid with KpnI. The DNA fragment was inserted into the KpnI site of pHSGX-K, to recover pKCTX-K. Cleaving pHSC4 with BamHI and KpnI, blunt ending both the termini of the resulting DNA fragment, followed by addition of a KpnI site, and directly inserting the resulting DNA fragment into the KpnI site of pHSGX-K, a plasmid of the same structure as that of pKCTX-K can be recovered.

### 〈2〉 Preparation of dtsR gene-disrupted strain

By homologous recombination process using a temperature-sensitive plasmid, dtsR gene-disrupted strain was recovered.

First, a defective-type dtsR gene for use in disrupting chromosomal dtsR genes was prepared. Inside a dtsR gene were present two sites digestible with Eco52I, namely positions 766 and 1366 in the SQ ID No.1 in the Sequence Listing. Therefore, pHSGX-K was completely digested with Eco52I, followed by self-ligation, to prepare plasmid pHSGX-KΔE containing a dtsR gene defective in the 600 base pairs as the Eco52I fragment. More specifically, the dtsR gene on pHSGX-KΔE has a structure defective in the central region in the frame.

By the following method, it was confirmed that the dtsR gene contained in pHSGX-KΔE did not function. A replication origin (Japanese Published Examined Patent Application No.109228/1995) derived from plasmid pHM1519 (K. Miwa et al., Agric. Biol. Chem., 48, 2901-2903 (1984)) autonomously replicable in coryneform bacteria was inserted into only one KpnI cleavage site present on pHSGX-KΔE. More specifically, pHSGX-KΔE was recovered, by digesting pHM1519 with restriction nucleases BamHI and KpnI to recover a gene fragment containing the replication origin, blunt ending the resulting fragment by using a blunting kit manufactured by TaKaRa Brewery, and subsequently inserting by using KpnI linker (manufactured by TaKaRa Brewery) the resulting fragment into the KpnI site of pHSGX-KΔE by using KpnI linker (manufactured by TaKaRa Brewery). As a control, pHSG399 (see Takeshita, S. et al.; Gene (1937), 61, 63-74; commercially available from TaKaRa Brewery) was used, and by using SalI linker (manufactured by TaKaRa Brewery), the replication origin of pHM1519 was inserted into the SalI site in the same manner, to prepare pSAC4. pKCX-KΔE and pSAC4 were individually inserted into a coryneform bacterial mutant strain with the increase in the sensitivity to detergents, namely Brevibacterium lactofermentum strain AJ11060, by using the electric pulse process, to examine the resistance to detergents. As to the method, 0 to 10 mg/deci-liter polyoxyethylene sorbitan monopalmitate was added to an M-CM2G liquid culture medium, to evaluate the individual growth. As a result, the defective-type dtsR gene lost the function to give resistance to detergents.

By inserting a DNA fragment containing TSori, which was recovered by cleaving pKCT4 with KpnI, into the KpnI cleavage site of pHSGX-KΔE, plasmid pKTCX-KΔE was prepared. By inserting pKTCX-KΔE into Brevibacterium lactofermentum ATCC 13869 as a wild type strain by using the electric pulse process, to substitute the dtsR gene on the chromosome with the defective type by the method described in Japanese Published Examined Patent Application No. 108228/1995. More specifically, ATCC 13869/pKTCX-KΔE was shaking cultured in an M-CM2G liquid culture medium containing 50 µg/ml oleic acid at 25 °C for 6 hours, which was then inoculated on an M-CM2G culture medium containing 5 µg/ml chloramphenicol and 50 µg/ml oleic acid, and a strain forming colonies at 34 °C was recovered as a plasmid-inserted strain. From the strain was then recovered a strain susceptible to chloramphenicol at 34 °C by replica method. The chromosome of the sensitive strain was recovered by a conventional method, followed by Southern hybridization to examine the structure of the dtsR gene on the chromosome and thereby verify that the dtsR gene was a defective type due to substitution, which was designated as strain ΔE. The recovery of the dtsR gene and the preparation of the dtsR gene-destroyed strain are described in detail in the pamphlet of the International Publication WO 95/23224.

The strain ΔE exerted a high potency to produce L-glutamic acid, but exerted a property to require oleic acid, and therefore, the strain could not grow in culture media with no oleic acid contained therein.

### 〈3〉 Preparation of a dtsR gene-destroyed strain carrying a temperature-sensitive plasmid containing dtsR gene

Temperature-sensitive plasmid pKCTX-K containing the complete length dtsR gene was inserted into the strain ΔE by the electric pulse process, to recover a strain ΔE/ pKCTX-K. Then, the culture was conducted at 25 °C, so that pKCTX-K might be retained in the cell.

### 〈4〉 Production of L-glutamic acid

Then, the growth between the strain ΔE and the strain ΔE/pKCTX-K was compared, together with their L-glutamate-producing potency. For seed culture and primary culture, 300-ml portions were divided in a 500-ml glass jar fermenter, and a culture medium sterilized under heating was used. The constituents of the culture medium are shown in Table 1. The pH of the culture medium was maintained at 7.3, by using ammonia gas. The aeration volume was 2/1 to 1/1 VVM, under agitation at 800 to 1300 rpm. Seed culture was conducted at culture temperatures shown in Table 2 for 13 to 14 hours: primary culture was conducted at culture temperatures shown in Table 3 for 35 hours. During the primary culture, 50 to 100 ml of an aqueous 50 g/liter glucose solution was fed. For culturing the strain ΔE, Tween 80 (polyoxyethylene sorbitan monopalmitate) as an oleic acid derivative was added at 1 mg/ml.

Tables 2 and 3 show the absorbance at 620 nm (OD620) of 51-fold diluted solutions of culture broths after culturing, the quantities of accumulated L-glutamic acid (in concentration g/dl) and the culturing periods of time.

**Table 1**

| Constituents of culture medium | Seed culture | Primary culture |
|---|---|---|
| Glucose (g/L) | 60 | 60 |
| KH₂PO₄ (g/L) | 1.5 | 1.5 |
| MgSO₄.7H₂O (g/L) | 1.0 | 1.5 |
| FeSO₄.7H₂O (g/L) | 0.01 | 0.015 |
| MnSO₄.7H₂O (g/L) | 0.01 | 0.015 |
| Soy bean milk concentrate*¹⁾ (ml/L) | 3.6 | 1.0 |
| Biotin (mg/L) | 0.45 | 0.5 |
| Vitamin B₁ (mg/L) | 0.45 | 0.2 |

| | | |
|---|---|---|
| *1): Acid hydrolysates of soy bean protein (with a total nitrogen at 3.49 g/100 mL) | | |

**Table 2**

| Bacterial strain | | Culture temperature (°C) | Tween80*¹⁾ | OD620 | Glu accumulation (g/dl) | Culturing period of time (h) |
|---|---|---|---|---|---|---|
| A | ΔE/pKCTX-K | 32 | non-addition | 2.00 | 0.2 | 13.5 |
| B | | 25→34*²⁾ | non-addition | 1.68 | 0.9 | 16.0 |
| C D | ΔE | 31.5 | added | 0.46 | 3.3 | 40.0 |
| | | 31.5 | added | -*³⁾ | - | - |

| | | | | | | |
|---|---|---|---|---|---|---|
| *1): denoting the addition or no-addition of Tween 80 (1 mg/ml). | | | | | | |
| *2): denoting temperature shift from 25 °C after 10-hr culture to 34 °C. | | | | | | |
| *3): denoting no growth. | | | | | | |

**Table 3**

| Seed culture*²⁾ | Culture temperature (°C) | Tween80*¹⁾ | Glu accumulation (g/dl) |
|---|---|---|---|
| A | 37 | no addition | 8.6 |
| B | 37 | no addition | 9.0 |
| C | 34 | added | 3.9 |

| | | | |
|---|---|---|---|
| *1): denoting the addition or no-addition of Tween 80 (1 mg/ml) | | | |
| *2): denoting the use of the bacteria recovered by the seed culture shown in Table 2. | | | |

The aforementioned results apparently indicate that the strain ΔE/pKCTX-K better grew with less production of L-glutamic acid, during seed culture, compared with the strain ΔE, and that the strain ΔE/pKCTX-K produced more L-glutamic acid. In other words, the strain ΔE/pKCTX-K grows well because the dtsR gene on the plasmid functions under culture conditions of low temperature and the strain produces L-glutamic acid well under culture conditions of high temperature because the dtsR gene is dissociated.

### (Example 2)

### Creation of an L-lysine-producing bacterium by utilizing HD gene and preparation of L-lysine by using the bacterium

### 〈1〉 Preparation of temperature-sensitive plasmid containing HD gene

### (1) Isolation of HD gene from coryneform bacteria

HD gene was isolated from Brevibacterium lactofermentum strain AJ12036 (FERM BP-734) in the following manner.

Because the nucleotide sequence of HD gene in Corynebacterium glutamicum was reported (Peoples, O. P. et al; Molecular Microbiology 2(1) 63-72 (1988)), and because it was estimated that the sequences of the individual HD genes of Brevibacterium lactofermentum and Corynebacterium glutamicum had a high similarity to each other, a synthetic primer DNA was prepared for use for PCR process, on the basis of the sequence of Corynebacterium glutamicum.

From Brevibacterium lactofermentum strain AJ12036 was prepared chromosomal DNA. So as to amplify an about-1500-bp DNA fragment containing HD gene from the chromosomal DNA by PCR process, two types of primers, namely 5'-side primer H1;
((841)5'-CTGGGAAGGTGAATCGAATT-3' (860): SQ ID No.5)
   and 3'-side primer H2;
((2410)5'-TCCGAGGTTTGCAGAAGATC-3' (2391): SQ ID No.6 )
were synthetically prepared, by using a DNA synthesizer of Model 1381A, manufactured by ABI, Co. The numerical figures in parenthesis show the positions in the nucleotide sequence presented by Peoples, et al (Peoples, O. P. et al; Molecular Microbiology 2(1) 63-72 (1988)) . The resulting synthetic primers were purified by reverse-phase HPLC.

PCR process was executed at the compositions shown in Table 4, by using a PCR amplification system (DNA Thermal Cycler PJ2000; manufactured by TaKaRa Brewery) and a PCR kit (TaKaRa GeneAmpTM kit; TaKaRa Brewery).

**Table 4**

| Constituents | Concentrations | Volumes blended |
|---|---|---|
| Primer H1 | 0.25 µM | 25pmol |
| Primer H2 | 0.25 µM | 25pmol |
| dATP,dGTP,dTTP,dCTP | 200 µM each | 20nmol |
| Taq DNA polymerase | 2.5U/100 µL | 0.5 µL(5U/µL) |
| Chromosomal DNA | | 1µg |
| 10× reaction buffer | | 10µL |
| Water | | balance (100 µL in total volume) |

During PCR, the reaction conditions for DNA denaturation, DNA annealing and polymerase reaction were 94 °C for one minute, 37 °C for 2 minutes, and 75 °C for 3 minutes, respectively, while the transfer time between the individual temperatures was one second. The reaction cycle was repeated 25 times, whereby DNA was amplified. The size of the resulting amplification reaction product thus recovered was confirmed by agarose gel electrophoresis, and as a result, it was observed that a DNA fragment of about 1.4 Kbp was amplified.

By inserting a DNA fragment prepared by cutting the thus recovered amplified fragment cleaved with KpnI into the KpnI site of vector plasmid pHSG399 (see Takeshita, S., et al.; Gene (1987), 61, 63-74); commercially available from TaKaRa Brewery), a recombinant plasmid was prepared, which was designated plasmid pHDW. The plasmid was inserted into an E. coli strain JM109, to recover a transformant.

The nucleotide sequence of the thus recovered HD gene fragment of the Brevibacterium lactofermentum strain AJ12036 was determined by the dideoxy method. The determined nucleotide sequence and putative amino acid sequence are shown as SQ ID Nos. 3 and 4, respectively, in the Sequence Listing.

### (2) Insertion of HD gene into a temperature-sensitive plasmid

The plasmid pHDW containing the HD gene was digested with KpnI, to cut out an HD gene fragment, which was then ligated to pHSG399 (manufactured by TaKaRa Brewery) after digestion with KpnI, by using T4 DNA ligase, to prepare plasmid pHDW.

Into the BamHI site of the thus recovered pHDW was inserted TSori. A DNA fragment containing TSori was prepared by cutting pHSC4 with BamHI and KpnI. Both the termini of the DNA fragment were blunt ended, followed by addition of a BamHI site, which was then inserted into the BamHI site of pHDW, to prepare pTSHD.

### 〈2〉 Preparation of HD gene-disrupted strain

An HD gene-destroyed strain was recovered, by homologous recombination using a temperature-sensitive plasmid.

First, a defective-type HD gene for use in gene destruction was prepared. Plasmid pHDW having the wild type HD gene was cleaved with AatII, which was then self-ligated together, to delete a region between the two AatII sites present within the HD gene (Nucleotide Nos. 716―721 and 1082―1087: SQ ID No.3), thereby preparing a plasmid containing a partially defective HD gene (HD-Δgene). By cleaving the plasmid with KpnI to prepare an HD-Δ gene fragment, which is then inserted in pHSG398 at the KpnI site, and by inserting a DNA fragment having TSori at the BamHI site, plasmid pTSHDΔ for substitution with ND-Δ gene was constructed. The DNA fragment having TSori was prepared from a plasmid modified so that it might be cleaved out solely with the cleavage only with BamHI, by blunt ending both the termini of a DNA fragment recovered by cleaving pHSC4 with restriction nuclease KpnI, by using a DNA blunting kit (manufactured by TaKaRa Brewery, Co.), and thereafter conjugating a BamHI linker (manufactured by TaKaRa Brewery) to the resulting termini.

By using the plasmid pTSHDΔ for substitution with the defective type HD gene Δ as recovered above, the transformation of Brevibacterium lactofermentum strain AJ11446 was carried out by the electric pulse method (Sugimoto et al., Japanese Published Unexamined Patent Application No.207791/1990). Herein, the strain AJ11446 is the L-lysine-producing bacterium described in Japanese Published Examined Patent Application No.24073/1987, which is deposited as Accession No. FERM P-5163 on date August 23, 1979 at the Life Engineering and Industrial Technology Research Institute, the Agency of Industrial Science and Technology (1-3, Higashi 1-chome, Tsukuba-shi, Ibaraki 305, Japan).

The resulting transformant strain was cultured by using M-CM2G culture medium at 25 °C until full growth (about 1-2×109/ml). The cultured bacteria were diluted to 105 cells per one plate and inoculated on an M-CM2G plate culture medium containing chloramphenicol (5 µg/ml), for culturing at 34 °C for 2 to 7 days, to recover colonies. It was confirmed that the cells in the resulting colonies did not contain the plasmid, and the integration of the plasmid for gene substitution into the chromosome was confirmed according to Southern hybridization using the linear pHSG398 as probe.

So as to leave only the defective type HD gene on the chromosome, the wild type HD gene and vector were dissociated from the chromosomal DNA, to obtain the mutant HD gene-substituted strain and the defective type HD gene-substituted strain. The dissociation of the wild type HD gene and vector was carried out as follows.

Each integrated strain was cultured in an M-CM2G culture medium containing chloramphenicol (10 µg/ml) at 34 °C until the strain reached full growth (1 - 2×109/ml). The cultured bacteria were inoculated at 50 to 200 colonies per one M-CM2G plate culture medium without chloramphenicol contained therein, for culturing at 34 °C. The grown colonies were replicated on an M-CM2G plate culture medium containing chloramphenicol (5 µg/ml) and then cultured at 34 °C to recover a chloramphenicol- susceptible strain. It was verified by Southern hybridization that the vector was dissociated from the chromosome of the resulting chloramphenicol-susceptible strain, and it was further confirmed that the defective type HD was expressed. Additionally, it was also confirmed that the resulting strain exerted methionine requirement and threonine requirement. The thus recovered gene-destroyed strain was defined as HDΔ strain. Based on the nucleotide sequencing of the chromosomal DNA of the HDΔ strain, it was verified that the gene was of defective type.

### 〈3〉 Preparation of HD gene-destroyed strain carrying a temperature-sensitive plasmid containing HD gene

By the electric pulse process, temperature-sensitive plasmid pTSHD containing the HD gene of the complete length was transfected into the HDΔ strain, to prepare a stain HDΔ/pTSHD. Culturing was then carried out at 25 °C in order that pTSHD might be retained in the cell.

### 〈4〉 Production of L-lysine

Subsequently, the strains HDΔ and HDΔ/pTSHD were compared with each other in terms of the growth and L-lysine productivity. For seed culture and primary culture, 300-ml portions were divided in a 500-ml glass jar fermenter, where a culture medium sterilized under heating was used. The constituents of the culture medium are shown in Table 5. The pH of the culture medium was maintained at pH 7.3, by using ammonia gas. Aeration was 2/1 to 1/1 VVM, and agitation was at 800 to 1300 rpm. Seed culture was carried out at the culture temperature as shown in Table 5 for 15 to 25 hours; primary culture was carried out at the culture temperature as shown in Table 6 for 30 hours. When intending to add L-methionine and L-threonine for cultivating the strain HDΔ, they were individually added at 70 mg/dl. For comparison, the parent strain AJ11446 was also cultured.

The culture broth after culturing was diluted 51-fold, to measure the absorbance at 620nm of the resulting solution as well as the quantity (concentration in g/dl) of the accumulated L-lysine, which are shown in Tables 6 and 7, together with the culturing period of time.

**Table 5**

| Constituents of culture medium | Seed culture | Primary culture |
|---|---|---|
| Glucose (g/L) | 50 | 150 |
| KH₂SO₄ (g/L) | 25 | 75 |
| KH₂PO₄ (g/L) | 1.0 | 1.0 |
| MgSO₄.7H₂O (g/L) | 1.0 | 1.0 |
| FeSO₄.7H₂O (g/L) | 0.01 | 0.01 |
| MnSO₄.7H₂O (g/L) | 0.01 | 0.01 |
| Soy bean milk concentrate*¹⁾ (ml/L) | 3.0 | 3.0 |
| Biotin (mg/L) | 0.45 | 0.45 |
| Nicotineamide (mg/L) | 0.5 | 0.5 |
| Vitamin B₁ (mg/L) | 0.2 | 0.2 |

| | | |
|---|---|---|
| *1): Acid hydrolysates of soy bean protein (at a total nitrogen of 3.49 g/100 ml) | | |

**Table 6**

| Bacterial strain | | Culture temperature (°C) | Met + Thr*¹⁾ | OD620 | Lys accumulation (g/dl) |
|---|---|---|---|---|---|
| E | HDΔ/pTSHD | 32 | non-addition | 0.82 | 1.7 |
| F | | 25→34*²⁾ | non-addition | 0.75 | 1.8 |
| G | HDΔ | 32 | added | 0.65 | 2.0 |
| H | | 32 | non-addition | -*³⁾ | - |
| I | AJ11446 | 32 | non-addition | 0.81 | 1.6 |

| | | | | | |
|---|---|---|---|---|---|
| *1): denoting the addition or non-addition of L-methionine and L-threonine (each 70 mg/mL). | | | | | |
| *2): denoting temperature shift to 34 °C after 10-hr culturing at 25 °C. | | | | | |
| *3) : denoting no growth. | | | | | |

**Table 7**

| Seed culture*²⁾ | Culture temperature (°C) | Met + Thr*¹⁾ | Lys accumulation (g/dl) |
|---|---|---|---|
| E | 34 | - | 6.8 |
| F | 34 | - | 7.2 |
| G | 34 | + | 6.2 |
| I | 34 | - | 5.9 |

| | | | |
|---|---|---|---|
| *1): denoting the addition or non-addition of L-methionine and L-threonine (each 70 mg/mL). | | | |
| *2): denoting that the bacterial strain recovered by the seed culture shown in Table 2 was used. | | | |

### (Example 3) Creation of an L-glutamate-producing bacterium by using α-KGDH gene and production of L-glutamic acid by using the same

### 〈1〉 Preparation of a temperature-sensitive plasmid containing α-KGDH gene

### (1) Isolation of α-KGDH gene

### (i) Preparation of a probe

Selecting a region with high homology in α-KGDH E1 subunit gene between Escherichia coli and Bacillus, oligonucleotides shown in SQ ID Nos. 9 and 10 in the Sequence Listing were synthetically produced by phosphoramidite process by using a DNA synthesizer (Model 394, manufactured by Applied Biosystems Inc.)

0.25 µmole each of the oligonucleotides as primers, 0.1 µg of the chromosomal DNA of Bacillus subtilis NA 64 (the strain was supplied from Bacillus Genetic Stock Center (Ohio State University, USA)) as prepared in a conventional manner as template and 2.5 units of Taq DNA polymerase (manufactured by TaKaRa Brewery) were added to 0.1 ml of 10 mM Tris-HCl buffer (pH 8.3) containing 200 µM each of dATP, dCTP, dGTP and dTPP, 50 mM potassium chloride, 1.5 mM magnesium chloride and 0.0001 % gelatin, and the resulting mixture was subjected to PCR for 30 cycles, each cycle consisting of 94 °C for one minute, 55 °C for 2 minutes and 72 °C for 3 minutes. The reaction solution was subjected to agarose gel electrophoresis, to recover the objective DNA fragment by using a glass powder (manufactured by TaKaRa Brewery). The DNA fragment was labeled according to a conventional labeling process using Klenow fragment (manufactured by Amersham, Co.) and [α-32P]-dCTP (manufactured by Amersham, Co.), which was then used as probe.

### (ii) Preparation of the chromosomal DNA of Brevibacterium lactofermentum ATCC 13869

Brevibacterium lactofermentum ATCC 13869 was inoculated and cultured in 500 ml of a T-Y culture medium, pH 7.2 comprising 1 % Bacto Tryptone (manufactured by Difco Ltd.), 0.5 % Bacto Yeast Extract (manufactured by Difco Ltd.) and 0.5 % sodium chloride at 31.5 °C for 6 hours, to obtain a culture. The culture was centrifuged at 5,000 rpm for 10 minutes, to recover wet bacteria of 2 g as the precipitate.

From the bacteria was extracted the chromosomal DNA according to the method by Saito and Miura (Biochem. Biophys. Acta., 72, 619 (1963)). 2 µg of the chromosomal DNA and 1,200 units of restriction nuclease EcoRI were independently mixed with 50 mM Tris-HCl buffer, pH 7.5 containing 10 mM magnesium chloride, 100 mM sodium chloride and 1 mM dithiothreitol, for reaction at a temperature of 37 °C for 15 hours. After the termination of the reaction, the solution was subjected to phenol extraction treatment in a conventional manner, followed by ethanol precipitation, to recover a fragment of the chromosomal DNA of the Brevibacterium lactofermentum ATCC 13869 after EcoRI digestion.

### (iii) Isolation of the α-KGDH gene of Brevibacterium lactofermentum ATCC 13869

One microgram of plasmid vector pUC18 (manufactured by TaKaRa Brewery) and 120 units of restriction nuclease EcoRI were mixed with 50 mM Tris-HCl buffer, pH 7.5 containing 10 mM magnesium chloride, 100 mM sodium chloride and 1 mM dithiothreitol, for reaction at a temperature of 37 °C for 2 hours, to recover a solution after digestion, and the solution was subjected to phenol extraction and ethanol precipitation in conventional manners. So as to prevent the reunion of the DNA fragments derived from the plasmid vector, the DNA fragments were thereafter dephosphorylated with bacterial alkaliphosphatase treatment according to the method described in Molecular Cloning, 2-nd edition, J. Sambrook, E. F. Fritsch and T. Maniatis, Cold Spring Harbour Laboratory Press, pl.60 (1989), followed by phenol extraction treatment and ethanol precipitation in conventional manners.

0.1 µg of pUC18 digested with EcoRI, 1 µg of the chromosomal DNA of the EcoRI-digested Brevibacterium lactofermentum ATCC 13869 as obtained in (ii) and 1 unit of T4 DNA ligase (manufactured by TaKaRa Brewery) were added to 66 mM Tris-HCl buffer, pH 7.5 containing 6.6 mM magnesium chloride, 10 mM dithiothreitol and 10 mM adenosine triphosphate, for reaction at a temperature of 16 °C for 8 hours to link the DNAs together. Then, Escherichia coli JM109 (manufactured by TaKaRa Brewery) was transformed with the DNA mixture, which was then inoculated on an L-agar culture medium containing 100 µg/ml ampicillin, to recover transformants of about 10,000 in number.

From the resulting transformants was selected a transformant hybridizable with the probe DNA recovered in (i), according to the method in Molecular Cloning, 2-nd edition, J. Sambrook, E. F. Fritsch and T. Maniatis, Cold Spring Harbour Laboratory Press, p1.90 (1989).

### (iv) Nucleotide sequencing of the α-KGDH gene of Brevibacterium lactofermentum ATCC 13869

From the transformant recovered it (iii) was prepared a plasmid DNA according to the alkali lytic process described in Molecular Cloning, 2-nd edition, J. Sambrook, E. F. Fritsch and T. Maniatis, Cold Spring Harbour Laboratory Press, p1.25 (1989). The plasmid DNA contained a DNA fragment of about 6 kilobase derived from the chromosomal DNA of Brevibacterium lactofermentum ATCC 13869. The plasmid was cleaved with restriction nucleases EcoRI and XhoI in the reaction composition described in (iii), followed by agarose gel electrophoresis in a conventional manner and Southern hybridization in the same manner as in (iii) to identity a fragment hybridizable with the probe DNA. As a result, it was demonstrated that an EcoRI-XhoI-cleaved fragment of about 3 kilobase was hybridizable. The DNA fragment was conjugated to pHSG397 (manufactured by TaKaRa Brewery) after digestion with EcoRI and XhoI as carried out in (iii), for cloning. By using the resulting plasmid DNA, the nucleotide sequence of the DNA fragment was determined. The determination of the nucleotide sequence was carried out by using Taq Dye Deoxy Terminator Cycle Sequencing Kit (manufactured by Applied Biochemicals Ltd.) according to the Sanger method (J. Mol. Biol., 143, 161 (1980)).

Because the resulting DNA fragment did not contain any complete open reading frame, the DNA fragment was transformed with a recombinant plasmid prepared by digesting the chromosomal DNA of Brevibacterium lactofermentum ATCC 13869 with XhoI, which was then conjugated to pHSG397, as carried out in (iii): by using as probe a fragment of about 3 kilobase, derived from the EcoRI/XhoI digestion of the chromosomal DNA of Brevibacterium lactofermentum ATCC 13869 as recovered in (ii) and after labeling according to the method in (i), a hybridizable transformant was selected. The plasmid carried in the resulting transformant, contained a DNA fragment of about 9 kilobase. A restriction map of a gene containing this DNA fragment is shown in Fig. 2. The plasmid was cleaved with restriction nucleases SalI and XhoI in the reaction composition in (iii), followed by agarose gel electrophoresis to identify a hybridizable fragment according to a conventional method, so that the fragment was a fragment of about 4.4 kilobase. As carried out in (iii), the DNA fragment was conjugated to plasmid vector pHSG397 after digestion with SalI and XhoI, for cloning. The plasmid was designated as pHSGS-X. The nucleotide sequence of a DNA fragment of about 1.4 kilobase, ranging from the SalI digestion site to the EcoRI digestion site in the SalI/XhoI digestion fragment contained in the plasmid, was determined in the same manner as described above.

The nucleotide sequence of the SalI-XhoI-digested gene fragment is shown as SQ ID No.7 in the Sequence Listing. The open reading frame was speculated, and the amino acid sequences of putative products based on the nucleotide sequence are shown as SQ ID Nos. 7 and 8. More specifically, the gene encoding the protein of the amino acid sequence of SQ ID No.8 is the α-KGDH gene of Brevibacterium lactofermentum ATCC 13869. It has been known that the methionine residue of the N terminus of the protein is derived from the ATG as initiation codon; that the methionine residue frequently does not have any relation with the function unique to the protein; and that the residue is removed via the function of peptidase after translation. The methionine residue may possibly be removed from the protein described above.

The nucleotide sequence and the amino acid sequence were individually compared with known sequences, in terms of homology. The database used were EMBL and SWISS-PROT. Consequently, the DNA shown as SQ ID No.7 and the protein encoded thereby were identified to be a protein with homology to the α-KGDH gene E1 subunit gene of Escherichia coli and Bacillus in previously reports, and the like.

The protein encoded by the present gene is composed of 1,257 amino acids inclusive of the methionine residue at the N terminus and has characteristic properties greatly different from those of the previously reported α-KGDH. In other words, amino acids of about 900 at the C terminus exerted high homology to various E1 subunits, but the 300 amino acids at the N terminus were never observed in the α-KGDHs of other species, which suggests that the present protein has specific functions. Compared with the region of the 300 amino acids at the N terminus with known sequences, in terms of homology, the region had homology to the E2 subunits of Escherichia coli and bacteria of genus Azotobacter. This indicates that the present protein may have the possibility to have both E1 and E2, unlike the α-KGDHs of other species.

Upstream the open reading frame of the present gene were observed a sequence (281-286 and 307-312) similar to the consensus sequence in promoters as observed in Escherichia coli and a sequence (422-428) similar to the ribosome-binding sequence of coryneform bacteria. Downstream the open reading frame of the present gene was observed a stem & loop structure (4243-4281) similar to the termination signal for transcription. These sequences suggest that the present gene is independently subjected to transcription and translation, having a gene structure different from those of the α-KGDHs of other species.

### (2) Insertion of α-KGDH gene in a temperature-sensitive plasmid

The pHSGS-X thus recovered as described above was cleaved with restriction nucleases BamHI and SalI, to recover a DNA fragment containing α-KGDH gene. The DNA fragment was ligated with plasmid pHSG299 (manufactured by TaKaRa Brewery) after the cleavage with BamHI and SalI by using T4 DNA ligase, to prepare pHSGS-X' .

TSori derived from coryneform bacteria was inserted in pHSGS-X' at the BamHI site. A DNA fragment containing TSori was prepared, by cleaving pHSC4 with BamHI and KpnI, blunt ending both the termini of the resulting DNA fragment, thereafter linking a BamHI linker to prepare plasmid pTBCT4, and thereafter cleaving the plasmid pTBCT4 with BamHI. The DNA fragment was inserted in pHSGS-X' at the BamHI site, to obtain plasmid pBKTS-X.

### 〈2〉 Preparation of α-KGDH gene-defective strain

An α-KGDH gene-destroyed strain was recovered by a homologous recombination process using a temperature-sensitive plasmid. More specifically, KpnI-digestible sites are present in the α-KGDH gene at two positions of 1340 and 3266 in SQ ID No.7 in the Sequence Listing. By partially digesting the resulting pHSGS-X recovered above with KpnI, followed by self-ligation, plasmid pHSGS-XΔK defective in the KpnI fragment of 1926 base pairs was prepared. The α-KGDH gene on the pHSGS-XΔK is of a structure defective in the central region. Then, TSori was inserted in pHSGS-XΔK at the BamHI recognition site, to prepare plasmid pBTS-XΔK. More specifically, pHSC4 was digested with restriction nuclease KpnI and then blunt ended by using a DNA blunting kit (manufactured by TaKaRa Brewery), to which a BamHI linker (manufactured by TaKaRa Brewery) was conjugated, followed by self-ligation, and the resulting plasmid was digested with restriction nuclease BamHI, to recover a gene fragment containing TSori, which was then inserted in pHSGS-XΔK at the BamHI site, to prepare plasmid pBTS-XΔK.

The plasmid was integrated in Brevibacterium lactofermentum ATCC 13869 as the wild type strain of an L-glutamate-producing coryneform bacterium by using the electric pulse process (Japanese Published Unexamined Patent Application No. 207791/1990), to substitute the α-KGDH gene on the chromosome with the defective type.

More specifically, plasmid-integrated ATCC 13869/pBTS-XΔK was shaking cultured in a CM2G liquid culture medium (1 % polypeptone, 1 % yeast extract, 0.5 % sodium chloride, 0.5 % glucose, pH 7.2) at 25 °C for 6 hours and subsequently, the resulting culture was inoculated in a CM2G agar culture medium containing 5 µg/ml chloramphenicol for culturing at 34 °C, and the resulting colonies were recovered as plasmid-integrated strains. From the strains, then, a strain susceptible to chloramphenicol was recovered at 34 °C by the replica method. In the susceptible strain, the nucleotide sequence of the α-KGDH gene on the chromosome was examined, so that it was confirmed that the α-KGDH gene was substituted with the defective type, and therefore, the strain was designated as ΔS strain. The α-KGDH activity of the strain ΔS was assayed, but no such activity was detected.

### 〈3〉 Preparation of an α-KGDH gene-disrupted strain carrying the temperature-sensitive plasmid containing α-KGDH gene

Temperature-sensitive plasmid pBKTS-X containing the complete length α-KGDH gene was integrated in the strain ΔS by the electric pulse process, to recover a strain ΔS/pBKTS-X. The culturing was then carried out at 25 °C so that pBKTS-X might be retained in the cells.

### 〈4〉 Production of L-glutamic acid

The strains ΔS and ΔS/pNKTS-X were compared with each other in terms of growth and L-glutamic acid producing potency. For seed culture and primary culture, 20 ml-portions were divided in Sakaguchi flasks; a culture medium sterilized under heating was used. The constituents of the culture medium are shown in Table 8. Seed culture was continued at a culture temperature shown in Table 9 until no sugar remained; primary culture was carried out under agitation at a temperature shown in Table 9 for 23 hours.

The culture broth after culturing was diluted 51-fold, and the absorbance at 620 nm (OD620)of the resulting solution and the quantity of accumulated L-glutamic acid (concentration in g/dl) are shown in Table 9, along with the amount (g/dl) of the remaining sugar.

**Table 8**

| Constituents of culture medium | Amounts blended |
|---|---|
| Glucose (g/L) | 30 |
| KH₂PO₄ (g/L) | 1.5 |
| MgSO₄.7H₂O (g/L) | 1.0 |
| FeSO₄.7H₂O (g/L) | 0.01 |
| MnSO₄.7H₂O (g/L) | 0.01 |
| Soy bean milk concentrate*¹⁾ (ml/L) | 4.8 |
| Biotin (mg/L) | 0.3 |
| Vitamin B₁ (mg/L) | 0.2 |
| 1M potassium phosphate buffer(pH 7.5)(ml) | 2.0 |

| | |
|---|---|
| *1): Acid hydrolysates of soy bean protein (with a total nitrogen of 3.49 g/100 ml) | |

**Table 9**

| Bacterial strain | Culture temperature (°C) | | OD620 | Glu accumulation (g/dl) | Residual sugar |
|---|---|---|---|---|---|
| | seed culture | primary culture | | | |
| | | | | | |
| ΔS/pBKTS-X | 25 | 25 | 1.024 | 0.00 | 0.0 |
| | 25→35*¹⁾ | 35 | 0.621 | 1.14 | 0.0 |
| ΔS | 25 | 25 | 0.063 | 0.29 | 2.7 |
| | 25→35*¹⁾ | 35 | 0.074 | 0.37 | 2.3 |

| | | | | | |
|---|---|---|---|---|---|
| *1): denoting temperature shift to 35 °C after 7-hr culturing at 25 °C. | | | | | |

The above results apparently indicate that compared with the strain ΔS, the strain ΔS/pBKTs-X grows well at 25 °C and produces more L-glutamic acid during primary culture. In other words, the strain ΔS/pBKTS-X grows well because the α-KGDH gene on the plasmid functions under culture conditions of low temperature but the strain turns an α-KGDH defective strain under conditions of high temperature so the strain efficiently produces L-glutamic acid.

### Industrial applicability

In the miciroorganism of the present invention, a gene disadvantageously functioning for the production of an objective enzyme and preferably advantageously functioning for the growth of a microorganism can be controlled at the retaining and dissociation extrachromosomally.

By culturing and growing a microorganism of the present invention, the objective substance can efficiently be produced.

## Claims

1. A microorganism containing a plasmid carrying a gene disadvantageously functioning for the production of an objective enzyme and a temperature-sensitive replication origin.

2. A microorganism according to claim 1, wherein the gene being functional is solely present on the plasmid.

3. A microorganism according to claim 1, wherein the gene is a gene encoding an enzyme belonging to another pathway branching from the biosynthetic pathway of the objective substance.

4. A microorganism according to claim 1, wherein the gene advantageously functions for the growth of the microorganism.

5. A microorganism according to claim 1, wherein the objective substance is an amino acid.

6. A microorganism according to claim 1, wherein the objective substance is L-glutamic acid and the gene is dtsR gene.

7. A microorganism according to claim 5, wherein the objective substance is L-glutamic acid and the gene is the gene of α-ketoglutaric acid dehydrogenase.

8. A microorganism according to claim 5, wherein the objective substance is L-lysine and the gene is the gene of homoserine dehydrogenase.

9. A microorganism according to any one of claims 1 to 8, wherein the microorganism is of coryneform bacteria.

10. A microorganism according to any one of claims 1 to 9, wherein the microorganism is recA-.

11. A method for producing an objective substance by a fermentation process, comprising a step of culturing and growing a microorganism according to any one of claims 1 to 10 at a temperature at which the plasmid is replicable, and a step of culturing the microorganism at a temperature at which the plasmid is never replicable to dissociate the plasmid from the cells to produce the objective substance.
